(19) Europäisches Patentamt / European Patent Office / Office européen des brevets

(11) **EP 4 674 423 A1**

(12) **EUROPEAN PATENT APPLICATION**
published in accordance with Art. 153(4) EPC

(43) Date of publication:
**07.01.2026 Bulletin 2026/02**

(21) Application number: 24762952.0

(22) Date of filing: **01.02.2024**

(51) International Patent Classification (IPC):
*A61K 36/64* (2006.01)      *A61K 36/258* (2006.01)
*A61K 9/14* (2006.01)      *A61K 9/20* (2006.01)
*A61P 21/04* (2006.01)

(52) Cooperative Patent Classification (CPC):
**A61K 9/14; A61K 9/20; A61K 36/258; A61K 36/64;
A61P 21/00; A61P 21/04; A61P 25/00**

(86) International application number:
**PCT/CN2024/075125**

(87) International publication number:
**WO 2024/179269 (06.09.2024 Gazette 2024/36)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **02.03.2023 CN 202310190284**

(71) Applicant: **Heibei Yiling Medical Research
Institute Co., Ltd
Shijiazhuang, Hebei 050035 (CN)**

(72) Inventor: **WU, Yiling
Shijiazhuang, Hebei 050035 (CN)**

(74) Representative: **V.O.
P.O. Box 87930
2508 DH Den Haag (NL)**

(54) **TRADITIONAL CHINESE MEDICINE COMPOSITION FOR TREATING AMYOTROPHIC
LATERAL SCLEROSIS AND USE THEREOF**

(57)      A traditional Chinese medicine composition for treating amyotrophic lateral sclerosis and the use thereof. The composition is prepared by means of combining Ginseng radix et rhizoma and Cistanches herba.

EP 4 674 423 A1

## Description

This application claims the priority of Chinese Patent Application No. 202310190284.4, filed with the China National Intellectual Property Administration on March 02, 2023, and titled with "TRADITIONAL CHINESE MEDICINE COMPOSITION FOR TREATING AMYOTROPHIC LATERAL SCLEROSIS AND USE THEREOF", the disclosure of which is hereby incorporated by its reference in entirety.

## FIELD

The present disclosure relates to the technical field of traditional Chinese medicine, in particular to a traditional Chinese medicine composition for treating amyotrophic lateral sclerosis and use thereof.

## BACKGROUND

Amyotrophic lateral sclerosis (ALS) is a neurodegenerative disease with unknown etiology, which primarily affects the motor neurons of the cerebral cortex, brainstem, and spinal cord. It is mainly manifested as progressive skeletal muscle weakness, atrophy, muscle fasciculation, bulbar palsy, and pyramidal tract signs in clinical. Usually, the survival period for patients with ALS is 3 to 5 years. Approximately 5 to 10% of cases are familial ALS (FALS) and 90 to 95% are sporadic ALS (SALS). The global incidence rate of ALS is approximately 1.59 people per 100,000 people, and the prevalence rate is 4.42 people per 100,000 people. In 2020, a big data analysis of medical insurance revealed that the total number of ALS patients in China was approximately 40,000, classifying it as a rare disease. This disease has an insidious onset, and the risk of its occurrence increases with age. It is a progressive and fatal disease. After some symptoms appear, the rate of deterioration is rapid. Currently, ALS, alongside cancer, acquired immune deficiency syndrome, leukemia, and rheumatoid arthritis, have been listed by the World Health Organization (WHO) as five major terminal illnesses. Diagnosing ALS clinically is difficult, leading to high rates of misdiagnosis and missed diagnosis. In addition, its treatment is costly. A survey on the economic burden of ALS patients in southwestern China revealed that the average annual total medical expenses per patient reaches 85,400 RMB, significantly higher than the national average annual income (35,128 RMB in 2021). As the disease progresses, ALS patients lose their self-care ability, placing a heavy burden on families and society. Therefore, there is an urgent need to find effective treatments and medications to slow disease progression and improve patients' quality of life.

The primary pathological manifestations of ALS include partial or complete disappearance of giant pyramidal cells in the bilateral precentral gyrus of the cerebral cortex, nuclear degeneration of motor neuron in the brainstem, degeneration of axons in central nervous system, and neurogenic muscle atrophy. Under normal physiological conditions, neurotransmitters synthesized in motor neurons are transported along axons to their terminals and then transmit excitatory signals to skeletal muscles via the neuromuscular junction, thereby causing excitation and contraction of skeletal muscles. When genetic mutations, glutamate excitotoxicity, oxidative stress, mitochondrial dysfunction, and other factors cause damage to motor neurons and dysfunction at the neuromuscular junction, the transmission by neurotransmitter is impeded, leading to muscle injury and the suppression of muscle contraction, which results in the onset of ALS. The pathogenesis of ALS involves lesions or damages at parts from presynaptic neuron to the neuromuscular junction, and these parts are interconnected and influence each other, which ultimately leads to damage primarily to the motor nervous system.

Rare diseases such as ALS should be diagnosed and treated early to extend the patient's survival period as much as possible. The "Guideline on clinical investigation of medicinal products for the treatment of amyotrophic lateral sclerosis (ALS)" released by European Medicines Agency propose that the main treatment goals for ALS are to improve survival rates, slow disease progression, and ameliorate ALS symptoms (muscle strength and related functions). The Guidelines for the Diagnosis and Treatment of Rare Diseases issued by the National Health Commission of China in 2019 recommends riluzole and edaravone for the treatment of ALS. Riluzole is a glutamate inhibitor, and its common adverse reactions include fatigue and nausea, and increased liver transaminases found in some individuals. On average, it can extend patients' survival period by approximately three months. However, the effects are not noticeable in the short term, and the dosing must be continued for over six months to improve prognosis. Edaravone is a free radical scavenger primarily indicated for early-stage patients with ALS whose courses of disease are less than two years and forced vital capacity (FVC) is greater than 80% of the predicted value. Its clinical benefits are limited, and clinical studies have confirmed that long-term injection of edaravone (8.9 to 13.9 months) does not ameliorate the condition. In 2022, a new combination formulation of sodium phenylbutyrate and taurursodiol was approved. Its clinical efficacy remains to be further validated. Common adverse reactions listed in the instruction of this formulation include diarrhea, abdominal pain, nausea, and infections of upper respiratory tract. A search with "amyotrophic lateral sclerosis" as the search term in the Pharmacodia database yielded a total of 247 records of drugs. Among them, only 1% have been approved for marketing: Masitinib, Tofersen, and Arimoclomol, 6% are in Phase III clinical trials, 13.4% are in Phase II clinical trials, 31.6% are in preclinical research, 9.7% are in a non-progressing state, and 11.3% have been terminated. Although Masitinib has been demonstrated to have an effect of delaying disease progression, its safety profile still requires further validation. Tofersen showed a trend toward reducing disease progression but failed to meet its primary endpoint.

According to the clinical symptoms such as decreased muscle strength and muscle atrophy, ALS is classified into the category of "Wei Syndrome" in Chinese medicine. The term "Wei" can refer to two aspects in the context of traditional Chinese medicine. In a first aspect, the term "Wei" refers to a disease name, also called as "lower limb weakness and flaccidity". This disease is classified into 5 types of Wei Syndrome in chapter *Wei Lun, Su Wen,* namely Skin-Flaccidity (Pi Wei), Channel-Flaccidity (Mai Wei), Sinew-Flaccidity (Jin Wei), Muscle-Flaccidity (Rou Wei) and Bone-Flaccidity (Gu Wei). In a second aspect, the term "Wei" refers to symptoms. Chapter *Yin Yang Bie Lun, Su Wen* points out that three Yin and three Yang diseases are manifested by hemiplegia, weakness and muscle flaccidity, and inability of limbs. Liu Hejian, in the *Suwen Xuanji Yuanbing Shi,* described that "the term "Wei" refers to the weakness and flaccidity of hands and feet causing the inability of movement". This means that "Wei" is a symptom of flaccidity and weakness of sinews and vessels of limbs, causing inability of movement. Regarding the pathogenesis of this disease, past medical practitioners primarily followed a general guiding principle recorded in *Inner Canon* that Wei is caused by zang-organs, and formed an understanding on pathogenesis of lung heat damaging fluids, dampness-heat exposure, deficiency and weakness of spleen and stomach, and deficiency of liver and kidney, and among them, sporadic discussions on treating Wei from the perspective of extra meridians, which provide valuable clinical experience for treating such refractory diseases.

*Inner Canon* and *Nan Jing* compiled in the Warring States period proposed that the Wei Syndrome has different patterns in terms of deficiency, excess and zang-organs, established the therapeutic principle of "treating Wei by focusing on the Yang-ming meridians", and also proposed that the Wei Syndrome is related to thoroughfare vessel, governor vessel and belt vessel, marking the inception of treating Wei Syndrome based on the extra meridians. During the Tang and Song dynasties, the view of treating Wei Syndrome based on deficiency and consumption of zang-fu organs led to the therapy centered on tonic medicines. During the Jin and Yuan dynasties, the critical roles of the spleen, stomach, liver and kidney in the pathogenesis of Wei Syndrome was emphasized, enriching pattern identification, syndrome differentiation, and treatment formulas. During Ming and Qing dynasties, medical practitioners formed the view of treating Wei Syndrome based on the deficiency and decline of Yuan-primordial qi, extra meridians and collaterals, and deficiency and consumption of great qi, further advancing traditional Chinese medicine research on Wei Syndrome. Past medical practitioners accumulated rich experience in treating Wei Syndrome from the perspective of zang-organs, and from interior and exterior factors and the pattern identification and analysis of deficiency and excess syndromes, they mainly focused on the view of the failure of nourishment to the sinews and muscles, slow action of sinews and vessels, and flaccidity and weakness of limbs which caused by lung heat damaging fluids, dampness-heat exposure, weakness of spleen and stomach, and deficiency of liver-kidney. As a result, the therapeutic principle such as "treating flaccidity by focusing on the Yang-ming meridians", clearing lung and moistening dryness, clearing and transforming dampness-heat, tonifying the spleen and stomach and tonifying the liver and kidney, and corresponding representative formulas were established. Particularly, therapies based on tonifying Yuan-primordial qi and nourishing the liver and kidney are predominant.

However, past medical practitioners only described the treatment of Wei based on extra meridians and collaterals in the *Inner Canon* and sporadic discussions during the Ming and Qing dynasties, but did not further develop or apply it. Consequently, for a long period in history, the effect of the extra meridians and collaterals on the development of this condition, and their role in such treatment, were not given adequate attention and systematically researched. Although renowned medical practitioners Ye Tianshi and Xu Yutai from the Qing Dynasty proposed that "both Sinew-Flaccidity and Bone-Flaccidity are attributed to extra meridians and collaterals" (See *Essence of Renowned medical Practitioners' Medical Records in Qing Dynasty·Xu Yutai),* these records lack detailed elaboration and fail to thoroughly investigate the mechanism. In reality, the eight extra meridians store qi and blood in twelve meridians and can govern waxing and waning of Yin and Yang aspects, qi and blood throughout the body. Starting from regulating the extra meridians and unblocking the collaterals is advantageous for elucidating the pathogenesis of this condition from the perspective of traditional Chinese medicine. This approach holds promise for exploring novel therapeutic strategies and is of significant importance for improving quality of life and ameliorating clinical symptoms of patients.

In summary, ALS is a rare disease that seriously threatens the lives of patients. Its clinical treatment faces serious problems such as a lack of treatment drugs, uncertain efficacy, obvious side effects, and difficulties in developing novel drugs. Therefore, the development of innovative drugs to effectively ameliorate clinical symptoms and improve quality of life is urgent and has become a major issue that needs to be addressed in clinical.

## SUMMARY

The academic viewpoint of "treating motor neuron disease based on extra meridians" proposed by Academician Wu Yiling provides a systematic theoretical guideline for various neuromuscular diseases, including ALS. The deficiency and poor nourishment of the eight extra meridians can cause the failure to warm, moisten and nourish zang-fu organs and twelve meridians of human body by qi, blood, Yin and Yang. The flaccidity is particularly closely related to the governor vessel. In addition, Wei Syndrome is also related to deficiency and consumption of zang-fu organs. The view of pattern identification based on zang-organs is thereby proposed. This means a comprehensive analysis of extra meridian dysfunction in connection with dysfunction of zang-fu organs according to the clinical symptoms and signs of ALS, which involves main pathogenesis such as deficiency and depletion of primordial essence, fluids deficiency of lung and stomach, scorched lung by heat, and stomach heat with Yin deficiency. A therapy of separate treatment of Sanjiao is established, wherein the suitable treatment of the upper jiao is ascending by clearing lung dryness, tonifying qi and generating fluids to support the lung's function of ascending, dispersing and distributing, or by supplementing qi and ascending sinking qi to assist the mediating function of great qi, or by tonifying heart blood to nourish all meridians and vessels; the suitable treatment of the middle jiao is transforming by strengthening spleen and assisting transportation to nourish the source of transformation and generation, or by clearing and transforming dampness-heat to unblocking the excess and stagnation in middle jiao; the suitable treatment of the lower jiao is supplementing by warming and regulating Yang of extra meridians to invigorate declined genuine primordial qi or by warming and supplementing genuine essence to enrich depleted Yin blood. These viewpoints provide novel strategies for treating Wei and hold significant guiding value for ALS treatment. Eight extra meridians, particularly the governor vessel, play an important and unique role in the treatment of Wei. Kidney essence in the governor vessel derives into the spinal cord and becomes the brain marrow upon entering into the brain. The governor vessel connects the kidneys and the brain via circulation therein and brings a thorough connection from the upper to lower body, coinciding with the pathologically affected parts in ALS. The innovative theory of, proposed by Academician Wu Yiling, "treating Wei based on extra meridians, pattern identification based on zang-organs, and separate treatment of Sanjiao" specifically treat Wei by targeting extra meridians, particularly governor vessel. This theory highlights the distinctive features of the eight extra meridians in traditional Chinese medicine theory while incorporating modern medical understanding of ALS. By integrating Chinese and Western medicine, it provides a clinically relevant and effective exploration of pathogenesis and therapeutic guidance.

Since the 1980s, Academician Wu Yiling has dedicated his research to the collaterals disease theory and its clinical applications. He classifies diseases with prolonged courses, recurrent onsets, migration and refractory characteristics as collateral diseases, with ALS being one of them. The collaterals disease theory points out that the collaterals as an intricate crisscross network branching out transversely from meridians, progressively subdivided, crisscrossing throughout the entire body and widely distributed across zang-fu organs and tissues, is a network structure that sustains biological activities and maintains the human body's internal homeostasis. Based on the functional distinction in circulating qi and blood, collaterals are classified into two types according to different functions of qi and blood, namely collaterals and vessels, thereby forming two branches of qi collateral theory and vessel collateral theory. The qi collateral theory specifies that while qi flows through meridians as qi of meridians, it enters the collaterals to become qi of collaterals. The qi of meridians diffuses via the qi collaterals to zang-fu organs, skin, muscles, limbs, and bones, exerting functions such as regulating, warming and nourishing, defending and protecting, transmitting information, and homeostaticallly regulating. Pathologies of the qi collaterals primarily involve disorders of the brain marrow and governor vessel, endocrine-metabolic dysfunctions, and immune abnormalities.

The qi collateral is a network structure that has a unique function and directly connects with the meridians and the brain marrow. As stated in the *Ling Shu: Xie Qi Zang Fu Bing Xing:* "The twelve meridians and the three hundred and sixty-five collaterals all transport blood and qi upward to the face and into the orifices". The collaterals also serve as essential pathways linking the extra meridians and brain marrow with muscles, sinews, and the limbs and all bones. The qi collateral is closely related to the governor vessel in the brain, forming an advanced neuroregulatory center in which the brain acts as the summit of qi movement (ascending and descending) and the spinal cord serves as the pathway for qi movement. This brain-spinal cord-qi collateral system constitutes a regulation mechanism of nervous system that regulates and controls zang-fu organs, and controls complex movement of external limbs.

The governor vessel is closely related to the spinal cord in both anatomical location and function from the perspective of modern medicine. Running along the midline of the back, the governor vessel overlaps with the spine, ascends to connect with the brain, and descends to link with the kidneys, thus serving as the "pathway for the ascending and descending of essence qi".

The brain's regulation function of movements depends on the governor vessel, which serves as the pathway for the ascending and descending of primordial essence and Yuan-primordial qi. The "fine collaterals" correspond closely to the description in modern neuroanatomy and histology, which is spinal nerves from the spinal cord progressively branch out to reach various internal and external parts of the body. The spinal cord aligns with the running of the governor vessel, it is therefore evident that and the fine collaterals branching from the spinal cord belong to the governor collaterals.

Apparently, Yuan-primordial qi transformed from the genuine essence in the kidneys moves along the governor vessel (spinal), and enters the collaterals in the brain. The function of the genuine primordial qi within the brain's qi collaterals reflects the action of brain spirit. Through the qi activity via governor collaterals, the brain spirit transmits its control and regulation effect downward to the zang-fu organs, limbs and all bones, thereby establishing a close relationship between the brain as well as spinal cord nerves and control and regulation of movements. The insufficiency of primordial essence, deficiency of genuine primordial qi, failure to nourish the governor collaterals, or failure to nourish qi collaterals may cause Wei ALS, as a group of neurodegenerative disorders of the central nervous system, is primarily pathologically characterized by the loss of pyramidal cells in the cerebral motor cortex, degeneration and loss of motor neurons in the anterior horn of the spinal cord and brainstem, abnormal pathological changes of cells, and degeneration of the corticospinal tract, thereby leading to muscle atrophy. Thus it can be seen that ALS lesions primarily occur at the sites of brain and spinal cord, which closely correspond to the running site of the brain marrow. The failure to nourish brain marrow due to the decline of genuine primordial qi and the deficiency of qi and Yang results in limb weakness, muscle atrophy, fatigue and laziness to speak. Therefore, ALS is classified as a disorder of the qi collateral-brain marrow-governor collateral system.

For the first time, proposed is that "the insufficiency of genuine primordial qi and failure of the governor collaterals to nourish" is a characteristic pathogenesis of ALS in traditional Chinese medicine. The insufficiency of genuine primordial qi is considered the root cause of the disease. Genuine primordial qi is Yuan-primordial qi transformed from primordial essence, circulates in the Sanjiao through meridians, and distributes across zang-fu organs. As the fundamental driving force of human life activities, genuine primordial qi activates the functional activities of the zang-fu organs by the regulation function of central nervous system. Since ALS lesions occur at the brain marrow, the insufficiency of innate essence directly leads to deficiency of both Yuan-primordial qi and primordial Yang. Additionally, due to essence qi being exhausted as aging, declined genuine primordial qi, and diminished functions of the zang-fu organs, ALS primarily affects middle-aged and elderly individuals. If further compounded by improper care, exposure to external pathogens, physical overwork, emotion disharmony, dietary irregularities, or sexual overindulgence, the damage to Yuan-primordial qi will be even more serious. The failure to nourish brain marrow causes its dysfunction, thereby leading to corresponding functional abnormalities. Therefore, the insufficiency of genuine primordial qi is considered as the root cause of this disease.

From the perspective of clinical manifestations, the deficiency of genuine primordial qi leads to the hypofunction of Yang in governing movement, vibrating, and ascending, and further causes inability of all systemic movement parts to movement, which is manifested as limb weakness, difficulty in walking, fatigue and laziness to speak, and shortness of breath. According to the theory of "Yang transforms into qi, and Yin forms physical appearance", the long-term Yang deficiency results in the incompetent Yin qi transformation. If the primordial Yang and Yuan-primordial qi distributed throughout the body are insufficient, they will be incapable of promoting and stimulating, sources for generation and transformation of qi and blood will be insufficient, and the dispersing will be impeded. These changes will result in the failure to nourish the muscles and skin, further in muscle weakness and atrophy. The deficiency of Yang qi causes the failure to warm and nourish sinews and meridians, which is manifested as increased muscle tone, muscle stiffness and the like. This disease presents syndromes including bulbar palsy and difficulty in swallowing due to the insufficiency of genuine primordial qi, clear Yang failing to ascend, turbid Yin failing to descend, disordered qi movement and impaired throat function.

Failure to nourish the governor vessel is the key to pathogenesis: the lesions of ALS primarily occur at the brain marrow, the governor vessel, one of extra meridians, just runs through bone marrow and brain, genuine primordial qi is derived from kidneys, and governed by the governor vessel, if the governor vessel and its collateral branches become deficient and the genuine primordial qi becomes insufficient, the effect by the governor vessel of governing and promoting Yang qi throughout the body will diminish, and it will particularly affect the running site of the governor vessel, leading to a failure to warm and nourish the spinal cord and brain and causing the occurrence of disease. The governor vessel runs up through the spine and neck and into the brain. A failure to fill the governor vessel leads to neck weakness, even waist weakness and ache in waist and knees. The deficiency and depletion of extra meridian and a failure to nourish the governor vessel weaken the effect of governing and promoting Yang qi throughout the body, leading to fatigue and laziness to speak. A failure to warm and nourish muscle leads to muscle twitching. A failure to fill the governor vessel, clear Yang failing to ascend and the dysfunction of upper orifices lead to dysarthria, difficulty in swallowing and choking when drinking water.

In summary, the warming, promoting, and nourishing functions of genuine primordial qi are fundamental to the normal functional activities of zang-fu organs. The governor vessel is connected to brain through the spine, and is considered as sea of Yang meridians. It exerts the effect of governing and guiding the activities of other meridians and zang-fu organs. The brain regulates the motor function of limbs by making the skin, muscles, sinews and bones subordinate to it through the movement of qi, i.e., its ascending and descending along the governor vessel and its collateral branches. If the genuine primordial qi is deficient and the governor collaterals losses its nourishment, the Yuan-primordial qi will not reach muscles, leading to the inability of muscles to move due to no activation by Yuan-primordial qi. The impairment and deficiency of the governor collaterals and the Yang deficiency of extra meridians weaken Yang qi throughout the body. This will particularly affect the running site of the governor vessel, leading to a failure to warm and nourish the spinal cord and brain and causing occurrence of disease. The pathogenesis above closely corresponds to the pathological features of ALS, including impairment of both upper and lower motor neurons and bulbar palsy. Therefore, understanding the pathogenesis of ALS from the perspective of extra meridians and the governor collaterals provides a new approach to therapeutic research.

A therapeutic method is established as reinforcing the Yuan-primordial qi to relieve Wei, and nourishing the nutrient qi to support the muscles. This method is designed to target the fundamental pathogenesis of ALS, namely deficiency of genuine primordial qi and failure to nourish the governor collaterals. Disorders of the extra meridians are often caused by the deficiency or depletion of Yuan-primordial qi. Therefore, the treatment thereof should take reinforcing the Yuan-primordial qi as the foundation. By reinforcing genuine primordial qi, Yuan-primordial qi and exuberant Yang qi can become exuberant, meridians and collaterals can be unblocked, and zang-fu organs and all bones can be nourished. Thus, *Ginseng Radix et Rhizoma* capable of supplementing Yuan-primordial qi is selected (1) to fill the governor vessel by nourishing and supplementing primordial Yang, (2) to achieve automatic essence generation and strengthening by supplementing qi in zang organs, and (3) to govern blood through qi movement, make qi and blood exuberant and unblock blood vessels by invigorating qi and unblocking blood vessels. After extra meridians are replenished and twelve meridians store sufficient qi and blood, muscles and interstices are nourished, achieving the effect of reinforcing the Yuan-primordial qi to relieve Wei. Therefore, it is suitable to use the herb capable of reinforcing Yin from Yang and Yang from Yin, and reinforcing the Yuan-primordial qi to relieve Wei, in combination with the herb capable of nourishing and supplementing genuine essence. This combination can nourish kidneys, supplement essence and blood, and achieve Yang generation as well as Yin supplement in herbs for nourishing and supplementing Yin essence, thereby achieving the purpose of reinforcing the Yuan-primordial qi to relieve flaccidity, and nourishing nutrient qi to support muscle by supporting Yuan-primordial qi, nourishing kidneys and supplementing essence.

The traditional Chinese medicine composition of the present invention consists of two herbs: *Ginseng Radix et Rhizoma,* and *Cistanche deserticola.* Wherein, *Ginseng Radix et Rhizoma* is sweet, slightly bitter and neutral, and enters spleen, lung and heart meridians. Its actions include greatly supplementing Yuan-primordial qi and nourishing qi collaterals. For the Wei Syndrome, the pathogenesis involves deficiencies of zang-fu organs, extra meridians, and the governor vessel. *Ginseng Radix et Rhizoma,* as sovereign medicine, can (1) treat the deficiency of eight extra meridians by greatly supplementing Yuan-primordial qi, nourishing and supplementing primordial Yang, (2) achieve automatic essence generation and strengthening of the body by supplementing Lung qi since when Lung qi is hyperactive, qi in other four zang-orangs is hyperactive, (3) govern blood through qi movement, make qi and blood exuberant and unblock blood vessels by invigorating qi and unblocking blood vessels. After extra meridians are replenished and twelve meridians store sufficient qi and blood, muscles and interstices are nourished, achieving the effect of reinforcing the Yuan-primordial qi to relieve Wei.

*Cistanche deserticola* is sweet and warm, has thick flavor, and enters kidney and large intestine meridians. Its actions include nourishing and supplementing kidney essence and nourishing nutrient qi to support muscle. This herb is warm but not dry and sweet-salty, and has an effect of moistening. It can supplement fire and support Yang without injuring Yin, efficiently treat five types of consumptions as well as seven types of damages and kidney deficiency with emaciation, which is a good herb for supplementing kidney essence and nourishing nutrient qi to support muscle. *Cistanche deserticola* can achieve Yang generation as well as Yin supplement in herbs for nourishing and supplementing Yin essence, making essence and blood sufficient, and thereby promoting the growth of muscles and warming the waist and knees. Thus, it can ameliorate syndromes including fear of cold and cold limbs and diminished muscle strength in patients with Wei Syndrome. *Cistanche deserticola* can tonifying kidney Yuan, supplement essence with warming and moistening, and nourish nutrient qi to support muscle. The combination of *Cistanche deserticola* and *Ginseng Radix et Rhizoma* as sovereign medicine can generate essence, supplement marrow, supplement qi, nourish blood and tonify Yang.

In summary, in this formula, *Ginseng Radix et Rhizoma* capable of reinforcing the genuine primordial qi and nourishing the governor vessel is used is in accordance with the principle of "physical weakness should be treated by warming qi", and *Cistanche deserticola* capable of nourishing and supplementing genuine essence, and nourishing nutrient qi to support muscle is used in accordance with the principle of "insufficiency of essence should be treated by nourishing with tonic herbs", respectively, to achieve the purpose of reinforcing the Yuan-primordial qi to relieve Wei, and nourishing nutrient qi to support muscle.

This formula is developed under the guidance of traditional Chinese medicine theory on clinical formula composition and based on clinical human data, reflecting the organic combination of modern bioinformatics and experimental techniques.

The main indication targeted by the traditional Chinese medicine composition of the present invention is amyotrophic lateral sclerosis due to the deficiency of Yuan-primordial qi in the governor vessel, which is manifested as decreased muscle strength, muscle atrophy, muscle twitching, muscle stiffness or flaccidity, dysarthria, difficulty in swallowing, shortness of breath, soreness and weakness of waist and knees, fatigue and laziness to speak, fear of cold and cold limbs, pale tongue, and weak pulse. The insufficiency of genuine primordial qi leads to the hypofunction of Yang in governing movement, vibrating, and ascending, and further causes inability of all systemic movement parts to move, which is manifested as limb weakness, difficulty in walking, fatigue and laziness to speak, and shortness of breath. The primordial Yang and Yuan-primordial qi distributed throughout the body are insufficient, which will be incapable of nourishing the muscles and skin, leading to muscle weakness and atrophy. The deficiency of Yang qi causes the failure to warm and nourish sinews and meridians, which is manifested as increased muscle tone, muscle stiffness and the like. The difficulty in swallowing is caused by the insufficiency of genuine primordial qi, clear Yang failing to ascend, turbid Yin failing to descend, disordered qi movement and impaired throat function. A failure to nourish the governor collaterals leads to neck weakness, even waist weakness and ache in waist and knees. A failure to fill the governor collaterals, clear Yang failing to ascend and the dysfunction of upper orifices leads to dysarthria, difficulty in swallowing and choking when drinking water. According to these views, proposed is a novel pathogenesis of ALS, namely "insufficiency of genuine primordial qi and failure to nourish the governor collaterals". The insufficiency of genuine primordial qi is considered the root cause of the disease, and the failure to nourish the governor collaterals is considered as key pathogenesis. Therefore, the therapeutic principle of "reinforcing the Yuan-primordial qi to relieve Wei, and nourishing nutrient qi to support muscle" is established. Based on this, the traditional Chinese medicine composition of the present invention is provided.

This formula fits the pathogenesis. In the traditional Chinese medicine composition of the present invention, *Ginseng Radix et Rhizoma* capable of greatly supplementing Yuan-primordial qi, nourishing qi collaterals and reinforcing the Yuan-primordial qi to relieve flaccidity is combined with *Cistanche deserticola* capable of nourishing and supplementing genuine essence, which can nourish kidneys, supplement essence and blood, and also achieve Yang generation as well as Yin supplement in herbs for nourishing and supplementing Yin essence, thereby achieving the purpose of reinforcing the Yuan-primordial qi to relieve Wei, and nourishing nutrient qi to support muscle by supporting Yuan-primordial qi, nourishing kidneys and supplementing essence. This prescription is an effective formula developed under the guidance of traditional Chinese medicine theory on clinical formula composition and based on the organic combination of clinical experience and data for human, modern bioinformatics and experimental techniques. It is designed to have a high degree of coherence among theory, methodology, formula, and herb, ensuring logical alignment with the disease's etiology, pathogenesis, therapeutic principles, formula-designing and herb-selecting, making the prescription rational.

To achieve the above-mentioned invention objectives, the present invention adopts the following technical solutions.

Provided by the present invention is a traditional Chinese medicine composition for use in treating amyotrophic lateral sclerosis, consisting of the following components in parts by weight: 3 to 9 parts of *Ginseng Radix et Rhizoma,* and 3 to 9 parts of *Cistanche deserticola*

Further, the traditional Chinese medicine composition consists of 4 to 6 parts of *Ginseng Radix et Rhizoma,* and 4 to 6 parts of *Cistanche deserticola.*

Further, the traditional Chinese medicine composition consists of 3 parts of *Ginseng Radix et Rhizoma,* and 9 parts of *Cistanche deserticola.*

Further, the traditional Chinese medicine composition consists of 6 parts of *Ginseng Radix et Rhizoma,* and 6 parts of *Cistanche deserticola.*

Further, the traditional Chinese medicine composition consists of 9 parts of *Ginseng Radix et Rhizoma,* and 3 parts of *Cistanche deserticola.*

Further, the traditional Chinese medicine composition consists of 7 parts of *Ginseng Radix et Rhizoma,* and 4 parts of *Cistanche deserticola.*

The present invention further provides a method for producing the traditional Chinese medicine composition, comprising producing an active ingredient of the traditional Chinese medicine composition by steps of: weighing *Ginseng Radix et Rhizoma* and *Cistanche deserticola* according to a prescribed amount, adding water, decocting, filtering to obtain a filtrate, and concentrating the filtrate under reduced pressure to obtain an extract as the active ingredient of the traditional Chinese medicine composition.

The present invention further provides use of the traditional Chinese medicine composition in the manufacture of a medicament for treating amyotrophic lateral sclerosis, wherein the amyotrophic lateral sclerosis is manifested as muscle weakness, muscle atrophy, muscle twitching, muscle stiffness or flaccidity, dysarthria, difficulty in swallowing, shortness of breath, soreness and weakness of waist and knees, fatigue and laziness to speak, or fear of cold and cold limbs.

The present invention further provides use of the traditional Chinese medicine composition in the manufacture of a medicament for delaying onset of paralysis in amyotrophic lateral sclerosis, improving impaired neurological function, alleviating sciatic nerve lesion, enhancing co-localization degree of neuromuscular junction.

The present invention further provides use of the traditional Chinese medicine composition in the manufacture of a medicament for prolonging hanging time for mice, increasing peak value of muscle action potential, shortening time course within region of interest.

The present invention further provides use of the traditional Chinese medicine composition in the manufacture of a medicament for ameliorating demyelination of central nervous system, increasing muscle strength and muscle fiber fullness, increasing blood supply to muscle tissue.

The present invention further provides use of the traditional Chinese medicine composition in the manufacture of a medicament for treating sarcopenia.

The present invention further provides use of the traditional Chinese medicine composition in the manufacture of a medicament for improving neuroelectrophysiology, reducing serum malondialdehyde level, promoting axonal growth and regeneration of myelin, protecting neurological function, enhancing co-localization degree of a muscular presynaptic marker and an acetylcholine receptor marker, increasing a number of positive satellite cells of gastrocnemius muscle and myelin basic protein (MBP) level, increasing structural integrity of neuromuscular junction, increasing expression of a neuronal marker (Tuj-1) and synaptic vesicle protein 2A (SV2A), promoting regeneration of myelin, improving behavioral change caused by demyelination, improving signal transmission from motor neuron to muscle, slowing progression of amyotrophic lateral sclerosis.

Further, the present invention provides a medicament comprising the above-mentioned traditional Chinese medicine composition or the traditional Chinese medicine composition produced by the above-mentioned method.

The traditional Chinese medicine composition of the present invention is in a dosage form of capsule, tablet, granule, injection, pill, powder, spray or oral liquid.

In order to realize the above dosage forms, pharmaceutically acceptable excipients should be added when producing these dosage forms, such as fillers, disintegrants, lubricants, suspending agents, binders, sweeteners, flavoring agents, preservatives, bases, and the like. The fillers include starch, pregelatinized starch, lactose, mannitol, chitin, microcrystalline cellulose, sucrose, and the like. The disintegrants include starch, pregelatinized starch, microcrystalline cellulose, sodium carboxymethyl starch, cross-linked polyvinylpyrrolidone, low-substituted hydroxypropyl cellulose, cross-linked sodium carboxymethyl cellulose and the like. The lubricants include magnesium stearate, sodium lauryl sulfate, talc, silicon dioxide and the like. The suspending agents include polyvinylpyrrolidone, microcrystalline cellulose, sucrose, agar, hydroxypropyl methylcellulose and the like. The binders include starch mucilage, polyvinylpyrrolidone, hydroxypropyl methylcellulose and the like. The sweeteners include sodium saccharin, aspartame, sucrose, sodium cyclamate, glycyrrhetinic acid and the like. The flavoring agents include sweeteners and various flavors. The preservatives include parabens, benzoic acid, sodium benzoate, sorbic acid and salts thereof, benzalkonium bromide, chlorhexidine acetate, eucalyptus oil and the like. The bases include PEG6000, PEG4000, insect wax and the like.

Further, the granule of traditional Chinese medicine composition of the present invention is produced by steps of:

(1) weighing *Ginseng Radix et Rhizoma and Cistanche deserticola* according to a prescribed amount, adding water, decocting to obtain a water extract, filtering the water extract to obtain a filtrate, and concentrating the filtrate to obtain an extract for later use;

(2) spray drying the extract using a spray dryer, and collecting spray powders for later use; and

(3) adding an appropriate amount of excipient to the spray powders obtained in step (2), and performing granulation according to a conventional method to obtain the granule.

Further, the granule of traditional Chinese medicine composition of the present invention is produced by steps of:

(1) weighing *Ginseng Radix et Rhizoma and Cistanche deserticola* according to a prescribed amount, adding water, decocting three times with 10 times the amount of water for a first decoction and 8 times the amount of water for each of a second and third decoctions, 1.5 h each time, filtering to obtain a filtrate, concentrating the filtrate under reduced pressure to a relative density of 1.05 to 1.10 at 60°C to obtain an extract for later use;

(2) spray drying the extract using a spray dryer, and collecting spray powders for later use; and

(3) adding an appropriate amount of excipient to the spray powders obtained in step (2), and performing granulation according to a conventional method to obtain the granule.

Further, the present invention further provides a method for treating a disease in a subject in need thereof, comprising administering the above-mentioned traditional Chinese medicine composition, the traditional Chinese medicine composition produced by the above-mentioned method or the above-mentioned medicament to the subject in need thereof.

The treating includes at least one of:

delaying the onset of paralysis in amyotrophic lateral sclerosis, improving impaired neurological function, alleviating sciatic nerve lesion, enhancing co-localization degree of neuromuscular junction;

prolonging hanging time for mice, increasing peak value of muscle action potential, shortening time course within region of interest;

ameliorating demyelination of central nervous system, increasing muscle strength and muscle fiber fullness, increasing blood supply to muscle tissue;

improving neuroelectrophysiology, reducing serum malondialdehyde level, promoting axonal growth and regeneration of myelin, protecting neurological function, enhancing co-localization degree of a neuromuscular presynaptic marker and an acetylcholine receptor marker, increasing a number of positive satellite cells of gastrocnemius muscle and myelin basic protein (MBP) level, increasing structural integrity of neuromuscular junction, increasing expression of a neuronal marker (Tuj-1) and synaptic vesicle protein 2A (SV2A), promoting regeneration of myelin, improving behavioral change caused by demyelination, improving signal transmission from motor neuron to muscle, and slowing progression of amyotrophic lateral sclerosis.

The method for producing the active ingredient of the traditional Chinese medicine composition provided by the present invention is simple, easy to operate, safe, and environmentally friendly, and can maximize the retention of the active ingredients in the traditional Chinese medicine to ensure the efficacy of the medicine.

## BRIEF DESCRIPTION OF DRAWINGS

FIG. 1 shows results of $EC_{50}$ of TN-2242 series compounds in glutamate-induced damage model.

FIG. 2 shows the effects of TN-2242 series compounds on the survival rate in glutamate-induced damage model (* indicates $P<0.05$).

FIG. 3 shows survival curves (not gender-specific).

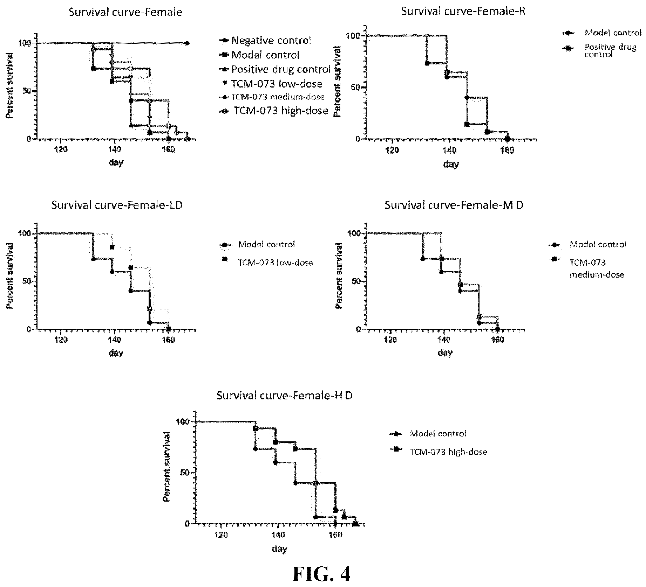

FIG. 4 shows survival curves (female).

FIG. 5 shows the image of HE staining ($20\times$) of soleus muscle in blank control group (female).

FIG. 6 shows the image of HE staining ($20\times$) of soleus muscle in blank control group (male).

FIG. 7 shows the image of HE staining ($20\times$) of soleus muscle in model control group (female).

FIG. 8 shows the image of HE staining ($20\times$) of soleus muscle in model control group (male).

FIG. 9 shows the image of HE staining ($20\times$) of soleus muscle in positive control group (female).

FIG. 10 shows the image of HE staining ($20\times$) of soleus muscle in positive control group (male).

FIG. 11 shows the image of HE staining ($20\times$) of soleus muscle in the low-dose group of the pharmaceutical composition of the present invention (female).

FIG. 12 shows the image of HE staining ($20\times$) of soleus muscle in the low-dose group of the pharmaceutical composition of the present invention (male).

FIG. 13 shows the image of HE staining ($20\times$) of soleus muscle in the medium-dose group of the pharmaceutical composition of the present invention (female).

FIG. 14 shows the image of HE staining ($20\times$) of soleus muscle in the medium-dose group of the pharmaceutical composition of the present invention (male).

FIG. 15 shows the image of HE staining ($20\times$) of soleus muscle in the high-dose group of the pharmaceutical composition of the present invention (female).

FIG. 16 shows the image of HE staining ($20\times$) of soleus muscle in the high-dose group of the pharmaceutical composition of the present invention (male).

FIG. 17 shows the image of HE staining ($10\times$) of gastrocnemius muscle in blank control group (female).

FIG. 18 shows the image of HE staining ($10\times$) of gastrocnemius muscle in blank control group (male).

FIG. 19 shows the image of HE staining (10×) of gastrocnemius muscle in model control group (female).

FIG. 20 shows the image of HE staining (10×) of gastrocnemius muscle in model control group (male).

FIG. 21 shows the results of immunofluorescence co-staining of synaptotagmin-2 as the presynaptic marker and α-BTX as the nicotinic acetylcholine receptor (nAChR) marker in the gastrocnemius muscle.

FIG. 22 shows the results of immunofluorescence co-staining of satellite cell marker Pax7 and Laminin 2α in the gastrocnemius muscle.

FIG. 23 shows the results of immunofluorescence staining of myelin basic protein (MBP) in the spinal cord.

FIG. 24 shows the results of immunofluorescence staining of neuronal marker Tuj-1 in the spinal cord.

## DETAILED DESCRIPTION

The content of the present invention will be described in further detail in combination with specific examples.

Example 1

Prescription: 600 of *Ginseng Radix et Rhizoma* and 600 g of *Cistanche deserticola*

Preparation Method:

The prescribed amounts of *Ginseng Radix et Rhizoma* and *Cistanche deserticola* were weighed, added with water and decocted three times. 10 times the amount of water was added for the first decoction and 8 times the amount of water was added for each of the second and third decoctions. Each decoction was carried out for 1.5 h. The resulting mixture was filtered to obtain a filtrate. The filtrate was concentrated under reduced pressure to a relative density of 1.05 to 1.10 at 60°C to obtain an extract. The extract was filtered for later use. The extract was subjected to spray drying using a spray dryer, with an inlet air temperature of 175°C±5°C and an outlet air temperature of 100°C±5°C. Spray powders were collected for later use. The spray powders and maltodextrin were mixed well and prepared into granules for later use. The granules were aliquoted at 5 g per sachet to obtain the final product.

Example 2

Prescription: 300 of *Ginseng Radix et Rhizoma* and 900 g of *Cistanche deserticola*

Preparation Method:

The prescribed amounts of *Ginseng Radix et Rhizoma* and *Cistanche deserticola* were weighed, added with water and decocted twice. 10 times the amount of water was added for the first decoction and 8 times the amount of water was added for the second decoction. Each decoction was carried out for 1.5 h. The resulting mixture was filtered to obtain a filtrate. The filtrate was concentrated under reduced pressure to a relative density of 1.05 to 1.10 at 60°C to obtain an extract. The extract was filtered for later use. The extract was subjected to spray drying using a spray dryer, with an inlet air temperature of 175°C±5°C and an outlet air temperature of 100°C±5°C. Spray powders were collected for later use. The spray powders and maltodextrin were mixed well and prepared into granules, which were encapsulated into capsules.

Example 3

Prescription: 900 of *Ginseng Radix et Rhizoma* and 300 g of *Cistanche deserticola*

Preparation Method:

The prescribed amounts of *Ginseng Radix et Rhizoma* and *Cistanche deserticola* were weighed, added with water and decocted three times. 8 times the amount of water was added for the first decoction and 10 times the amount of water was added for each of the second and third decoctions. Each decoction was carried out for 2 h. The resulting mixture was filtered to obtain a filtrate. The filtrate was concentrated under reduced pressure to a relative density of 1.05 to 1.10 at 60°C to obtain an extract. The extract was filtered for later use. The extract was subjected to spray drying using a spray dryer, with an inlet air temperature of 175°C±5°C and an outlet air temperature of 100°C±5°C. Spray powders were collected for later use. The spray powders and maltodextrin were mixed well and prepared into granules. The granules were prepared into tablets according to a conventional method.

Example 4

Prescription: 700 of *Ginseng Radix et Rhizoma* and 400 g of *Cistanche deserticola*

Preparation Method:

The prescribed amounts of *Ginseng Radix et Rhizoma* and *Cistanche deserticola* were weighed, added with water and decocted twice. 10 times the amount of water was added for each decoction. Each decoction was carried out for 2 h. The resulting mixture was filtered to obtain a filtrate. The filtrate was concentrated under reduced pressure to a relative density of 1.05 to 1.10 at 60°C to obtain an extract. The extract was filtered and prepared into an oral liquid according to a conventional method.

Example 5

600 of *Ginseng Radix et Rhizoma* and 600 g of *Cistanche deserticola*

*Ginseng Radix et Rhizoma* and *Cistanche deserticola* were added with 10 times the amount of water and decocted twice. The resulting mixture was filtered and concentrated under reduced pressure to obtain an extract. The extract was dried to obtain spray powders.

Comparative example 1

1200 g of *Ginseng Radix et Rhizoma*

*Ginseng Radix et Rhizoma* was added with 10 times the amount of water and decocted twice. The resulting mixture was filtered and concentrated under reduced pressure to obtain an extract. The extract was dried to obtain spray powders.

Comparative example 2

1200 g of *Cistanche deserticola*

*Cistanche deserticola* was added with 10 times the amount of water and decocted twice. The resulting mixture was filtered and concentrated under reduced pressure to obtain an extract. The extract was dried to obtain spray powders.

Comparative example 3

860 g of *Ginseng Radix et Rhizoma* and 340 g of *Epimedii Folium*

*Ginseng Radix et Rhizoma* and *Epimedii Folium* were added with 10 times the amount of water and decocted twice. The resulting mixture was filtered and concentrated under reduced pressure to obtain an extract. The extract was dried to obtain spray powders.

Comparative example 4

600 g of *Ginseng Radix et Rhizoma* and 600 g of *Epimedii Folium*

*Ginseng Radix et Rhizoma* and *Epimedii Folium* were added with 10 times the amount of water and decocted twice. The resulting mixture was filtered and concentrated under reduced pressure to obtain an extract. The extract was dried to obtain spray powders.

Comparative example 5



1000 of *Ginseng Radix et Rhizoma* and 200 g of *Cistanche deserticola*

*Ginseng Radix et Rhizoma* and *Cistanche deserticola* were added with 10 times the amount of water and decocted twice. The resulting mixture was filtered and concentrated under reduced pressure to obtain an extract. The extract was dried to obtain spray powders.

Comparative example 6

200 of *Ginseng Radix et Rhizoma* and 1000 g of *Cistanche deserticola*

*Ginseng Radix et Rhizoma* and *Cistanche deserticola* were added with 10 times the amount of water and decocted twice. The resulting mixture was filtered and concentrated under reduced pressure to obtain an extract. The extract was dried to obtain spray powders.

Experimental example 1: Study on the intervention effects of various extracts and combinations thereof on the ALS-related cell model

1. Experimental objective

This study used rat pheochromocytoma PC-12 cells damaged by glutamate as a model to evaluate the intervention effects of various extracts and combination groups on the cell viability of the PC-12 cell damage model induced by glutamate, to confirm the extracts or combination groups for further investigation.

2. Experimental materials

2.1 Samples to be screened

| Number of Test product | Test Product | Content | Batch Number |
|---|---|---|---|
| TN-2242-1 | The pharmaceutical composition of the present invention (Example 5) | 1.99 g of raw drug/g drug powder | 220325-1 |
| TN-2242-2 | Comparative example 1 | 2.06 g of raw drug/g drug powder | 220325-2 |
| TN-2242-3 | Comparative example 2 | 2.71 g of raw drug/g drug powder | 220325-3 |
| TN-2242-4 | Comparative example 3 | 2.63 g of raw drug/g drug powder | 220325-4 |
| TN-2242-5 | Comparative example 4 | 2.66 g of raw drug/g drug powder | 220325-5 |
| TN-2242-6 | Comparative example 5 | 2.25 g of raw drug/g drug powder | 220325-6 |
| TN-2242-7 | Comparative example 6 | 2.50 g of raw drug/g drug powder | 220325-7 |
| Source | Shijiazhuang Yiling Pharmaceutical Co., Ltd. | | |
| Expiration date | Tentatively set as March 24, 2023 | | |
| Storage | Normal temperature, dryness | | |
| condition | | | |

2.2 Reagents and tools

2.2.1 Modeling agent: L-glutamic acid
2.2.2 Reagents and kits: DMEM medium, DMSO, trypsin, double-antibiotics, fetal bovine serum, PBS, MTS assay kit

2.3 Experimental system

2.3.1 Cell line and source
Rat pheochromocytoma PC-12 cells frozen in in-house cell laboratory

2.3.2 Instrument system

SW-CJ-1FD Clean Bench (Suzhou Antai Airtech Co., Ltd.)
Protect-2FD-S Clean Bench (Thermo Fisher Scientific Inc. (China))
Olympus CKX53 Microscope (Olympus)
Thermo3111 $CO_2$ incubator (Thermo Fisher Scientific Inc.)
Infinite M200 PRO Microplate Reader (TECAN, Switzerland)
AMQAX1000 Cell Counter (Thermo Fisher Scientific Inc.)
DK-98-II Electric-heated Thermostatic Water Bath Pot (Tianjin Taisite Instrument Co., Ltd.)
Sorvall ST 16/16R High-Speed Freezing Centrifuge (Thermo Fisher Scientific Inc.)
BPN-CH $CO_2$ Incubator (Shanghai Yiheng Scientific Instrument Co., Ltd.)

3. Experimental methods

3.1 Preparation of main reagents:

Each extract and its combination were dissolved in DMSO to prepare a 500 mg/ml solution and stored at -20°C for later use.

3.2 Experimental grouping and intervention methods:

Cells in the logarithmic growth phase were seeded into the cell culture plate at a cell density of $1 \times 10^5$ cells/mL. When the cells reached 80% confluence, the culture medium was discarded and the cells were divided into the following groups (6 replicate wells were set for each group).

3.2.1 Experimental grouping of samples to be screened

(1) Blank control group (Control): Cells were cultured with a serum-free medium.

(2) Model group (Model): Cells were cultured with a serum-free medium containing 20 mM glutamate.

(3) Solvent group (DMSO): Cells were added with a serum-free medium containing 0.1% DMSO and 20 mM glutamate and cultured.

(4) Drug group: Each drug was added to cells at concentrations of 500 μg/ml, 50 μg/ml and 5 μg/ml, respectively, and a serum-free medium containing 20 mM glutamate was added to cells for culture.

4. Detection indicator

Cell Viability

Cell viability was detected using a 96-well plate and MTS assay reagents, and the operation procedure was carried out strictly in accordance with the kit instructions.

5. Statistical methods

Experimental data are presented as mean $\pm$ standard deviation ($\overline{x}\pm s$). $EC_{50}$ values were calculated by using GraphPad Prism 8 software, and statistical analysis was performed using T-test.

6. Results

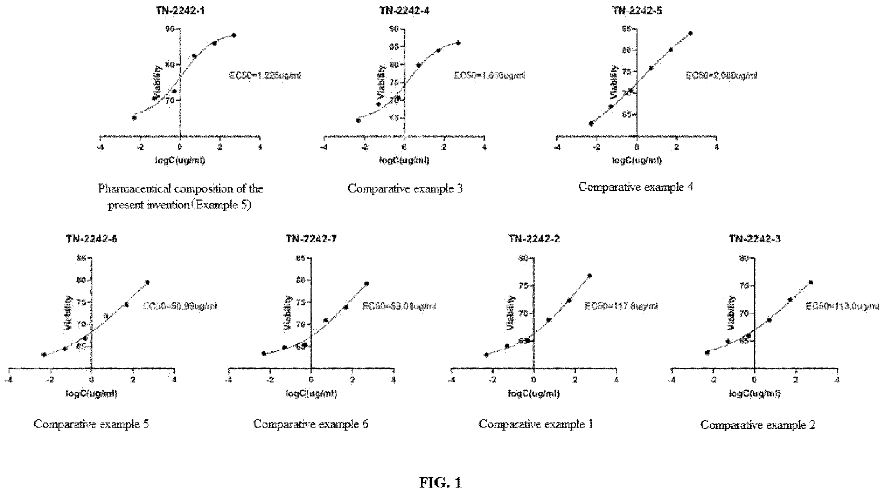

TN-2242 series compounds were added to glutamate-damaged PC12 cells at concentrations of 500 $\mu$g/ml, 50 $\mu$g/ml, 5 $\mu$g/ml, 500 ng/ml, 50 ng/ml, and 5 ng/ml, respectively. After 24 hours, the MTS assay was carried out to detect the survival rate of cells. The $EC_{50}$ value for the inhibition of each compound on the glutamate-induced damage to nerve cells was calculated. The results are shown in FIG. 1. Among them, the $EC_{50}$ value of the pharmaceutical composition of the present invention was 1.225 $\mu$g/ml, which was the lowest. The $EC_{50}$ values of the other extracts and combination groups are shown in Table 1.

Table 1 $EC_{50}$ Value of TN-2242 series compounds in glutamate-induced damage model

| Name | TN-2242-1 | TN-2242-2 | TN-2242-3 | TN-2242-4 | TN-2242-5 | TN-2242-6 | TN-2242-7 |
|---|---|---|---|---|---|---|---|
| $EC_{50}$ ($\mu$g/ml) | 1.225 | 117.8 | 113 | 1.656 | 2.08 | 50.99 | 53.01 |

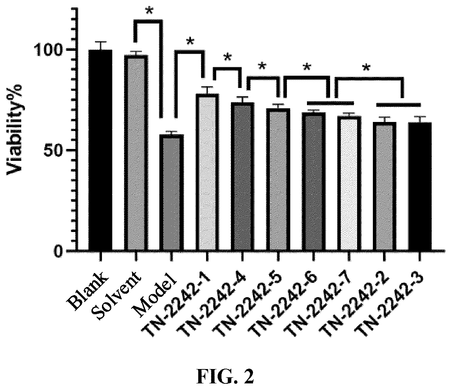

Subsequently, referring to the $EC_{50}$ value of the pharmaceutical composition of the present invention, the protective effect of each extract and combination at a concentration of 1 $\mu$g/ml on glutamate-damaged PC12 cells was tested. The results (FIG. 2 and Table 2) show that the protective effect of the pharmaceutical composition of the present invention is superior to that of comparative examples 1-6.

Table 2 Survival rate of glutamate-induced damage model treated with TN-2242 series compounds

| | Blank control | Solvent control | Model | TN-2242-1 | TN-2242-2 | TN-2242-3 | TN-2242-4 | TN-2242-5 | TN-2242-6 | TN-2242-7 |
|---|---|---|---|---|---|---|---|---|---|---|
| | | | | 1$\mu$g/ml | | | | | | |
| %Survival rate | 100$\pm$3.5 | 97.32$\pm$1.66 | 57.70$\pm$1.43 | 78.16$\pm$3.00 | 64.01$\pm$2.19 | 63.95$\pm$2.57 | 73.87$\pm$2.34 | 70.90$\pm$1.76 | 68.84$\pm$1.04 | 67.14$\pm$1.32 |

The above test results show that the protective effect of the pharmaceutical composition of the present invention is superior to that of other extracts and combinations in the glutamate-induced damage model.

7. Record and storage

Except for data collected directly by computers or automated instruments, all other data obtained in actual research were recorded in forms or record sheets and organized and bound in time.

8. Data archiving

8.1 Storage unit: Innovative Drug Evaluation Center of Hebei Yiling Pharmaceutical Research Institute

8.2 Address: No. 238, Tianshan Street, High-Tech Development Region, Shijiazhuang

Experimental example 2: Effect of the pharmaceutical composition of the present invention on the survival period of SOD1-G93A transgenic mice with amyotrophic lateral sclerosis

Experimental objective

By using SOD1-G93A transgenic mice, the therapeutic effect of the pharmaceutical composition of the present invention on amyotrophic lateral sclerosis was evaluated.

1 Experimental materials

1.1 Test products

1.1.1 Name: The pharmaceutical composition of the present invention prepared in Example 1 and abbreviated as TMC-073 (test product number: GLPTN-2106).
1.1.2 Characteristics: The product is a yellowish-brown powder, slightly fragrant and slightly bitter.
1.1.3 Functions and Indications: amyotrophic lateral sclerosis.
1.1.4 Clinical dosage and administration route: daily dose: 18 g of raw drug per day, oral administration.
1.1.5 Specification: 3.1 g of raw drug/g powder.
1.1.6 Source: Shijiazhuang Yiling Pharmaceutical Co., Ltd. Batch number: 210701.
1.1.7 Storage condition: normal temperature and dryness.
1.1.8 Expiration date: tentatively set as July 27, 2022.

1.2 Positive drugs and main reagents

1.2.1 Positive drugs: Riluzole (brand name: Rilutek): 50 mg per tablet. Riluzole is indicated for the treatment of amyotrophic lateral sclerosis. It can extend survival period for patients and/or delay the need for tracheostomy. For adults, an oral administration of 50 mg (one tablet) twice daily is adopted in clinical. It was manufactured by Sanofi Winthrop Industrie and packaged by Sanofi (Beijing) Pharmaceuticals Co., Ltd. Batch Number: 0KP4C. Expiration date: May 2023.
1.2.2 Main reagents: Sterile water for injection: Shijiazhuang No. 4 Pharmaceutical Co., Ltd. Batch Number: 1911143205 with Expiration date: 2021.11.13. Batch Number: 2007023202 with Expiration date: 2022.07.01.

1.3 Experimental system

1.3.1 Animal Strain: B6SJL-Tg (SOD1-G93A) 1Gur/J (Stock Number: 002726) was provided by The Jackson Laboratory (United States). Maintaining and breeding strain, and identifying the offspring of SOD1-G93A transgenic mice were conducted by Jiangsu GemPharmatech Co., Ltd. Littermates that did not carry the mutant human SOD1 gene were used as the negative control.
1.3.2 Animal Grade: SPF grade.
1.3.3 Animal Gender and Quantity: One-hundred and fifty B6SJL-Tg (SOD1-G93A) 1Gur/J mice. Twenty littermates that did not carry the mutant human SOD1 gene were used. Both males and females were included.
1.3.4 Animal Age: 53 days old.
1.3.5 Animal Weight: male mice (22.8-34.4 g), and female (16.9-25.3 g).
1.3.6 Animal Source: Purchased from The Jackson Laboratory (United States). Breeding was conducted by Jiangsu GemPharmatech Co., Ltd.

1.3.7 Animal Certification of Quality, issuing authority, and receipt date: Certification number 320727210100625138. License number SCXK (Jiangsu) 2018-0008. Issuing authority: Jiangsu GemPharmatech Co., Ltd. Receipt date: August 11, 2021.

1.3.8 Feeding Conditions: Mice were reared in cages at the Innovative Drug Evaluation Center of Hebei Research Institute of Integrated Traditional Chinese and Western Medicine. License Number: SYXK (Hebei) 2020-006. Lighting: 12 h/day. Temperature: 20-26°C. Relative Humidity: 40%-70%.

1.3.9 Quarantine Process: Animals were subjected to quarantine for 5 days. During this period, whether the animals had normal water and food intake, and whether the animals were in good health status, had illness and showed signs of mortality were observed.

1.3.10 Feed: Rat and mice maintenance feed (SPF-grade) was provided by Beijing Keao Xieli Feed Co., Ltd. Feed Production License Number: Beijing Feed Certificate (2018) 06073. Experimental Animal Production License Number: SCXK (Beijing) 2019-0003. Product Batch Number: 21033213 with Expiration date: 2021.08.31. Product Batch Number: 21073213 with Expiration date: 2022.01.03. Product Batch Number: 21093213 with Expiration date: 2022.03.03.

1.3.11 Water: Clean water prepared by the ROB-B drinking water system for laboratory animal was provided, and filled in water bottles for free drinking. The drinking water was detected annually for all indicators, mainly including microbiological indicators, toxicological indicators, sensory characteristics, and general chemical parameters, and the detection was commissioned to a qualified institution. Microbial limit detection was performed every 2 months, which was commissioned to the Quality Control Department of Shijiazhuang Yiling Pharmaceutical Co., Ltd. The detection reports were stored by the animal department and archived annually.

1.3.12 Bedding: Corn cob bedding (common grade) provided by Beijing Keao Xieli Feed Co., Ltd., was sterilized by high temperature and high pressure before use. Batch Number: 21099811.

1.3.13 Marking: Mice were marked with ear tags, provided by Globalebio (Beijing) Technology Co., Ltd.

2 Test methods

2.1 Test design basis

2.1.1 Adopted standards were determined according to the Registration, Classification and Submission Data Requirements for Chinese Traditional Medicine and Natural Drugs issued by the State Food and Drug Administration, Good Laboratory Practice for non-clinical laboratory studies, and related literatures.

2.1.2 Description of selected experimental system: Currently, the transgenic B6SJL-Tg(SOD1-G93A)1 Gur/J mice carrying a mutant human Cu/Zn superoxide dismutase (SOD) gene is considered the most commonly used and best animal models for amyotrophic lateral sclerosis (ALS). The pathogenesis of ALS remains largely unknown, with 90% of cases being sporadic and only 10% familial (FALS). In 1993, Rosen found missense mutations in the SOD1 gene, which was a significant milestone in ALS research. The identification of SOD1 mutations in FALS brought the subsequent development of animal models for ALS. Nowadays, the SOD1-G93A mice are widely used as the transgenic mice model for ALS both in clinical and basic research, which carry high copies of mutant human SOD1 gene. Homozygous mice exhibit a reduction in spinal motor neurons and gradually exhibit neuromuscular dysfunction at around 3 months after birth, including limb tremor under tail suspension and hemiplegia of one or more limbs. Their lifespan typically is about 4-5 months. This model can effectively mimic the chronic progressive course of ALS along with some pathological and biochemical characteristics of the disease.

2.2 Dosage and grouping

After ALS model mice were determined to have significant changes compared to negative control mice in behavioral test by combining literature as well as prior experience, they were randomly grouped according to their body weight. The drug administration was started and mice were observed. The groups were specifically divided into: model control group (M), positive drug control group (Riluzole, 0.02 g/kg, R), and low-dose (073-L), medium-dose (073-M), and high-dose groups (073-H) of the pharmaceutical composition of the present invention, with 30 animals in each group. Additionally, wild-type littermates that did not carry mutant human SOD1 gene were used as the negative control group (C; a total of 20 mice). Specific experimental groupings and doses are shown in Table 3.

2.3 Administration method

The drug was orally administered via gavage, consistent with the clinically recommended oral administration.

2.4 Preparation and storage of test product

The clinical dose of Riluzole was calculated as approximately 0.002 g/kg based on a body weight of 60 kg. The administration dose for mice was 10 times the clinical dose , approximately 0.02 g/kg. The administration volume was 10 ml/kg and administration concentration was 0.02 g/10 ml. The drug was prepared using powder obtained by grinding the tablets, with each tablet containing 50 mg of Riluzole. During the preparation, one tablet was taken, ground with sterile injectable water and adjusted to a final volume of 25 ml.

The proposed clinical dose of the pharmaceutical composition of the present invention was 18 g of raw drug/day. This test set three doses: low dose (1.5 g raw drug/kg), medium dose (3.0 g raw drug/kg), and high dose (6.0 g raw drug/kg), corresponding to 5, 10, and 20 times the clinical dose, respectively (See Table 3 for details). An appropriate amount of the drug was weighed and ground using sterile injectable water as the solvent to prepare high, medium, and low doses.

Table 3 Experimental grouping and dose setting

| Group | | Number of animals | Dose (g raw drug/kg) | Specification(g raw drug/g powder) | Dose for formulation (g powder/kg) | Formulation concentration (g powder/ml) | Multiple relative to clinical equivalent dose |
|---|---|---|---|---|---|---|---|
| Negative control group | | 20 | - | - | - | - | - |
| Model control group | | 30 | - | - | - | - | - |
| Positive drug control group (Riluzole) | | 30 | 0.02 g/kg | 50mg/tablet | - | 25ml/tablet | 10 |
| TCM-073 | Low-dose | 30 | 1.50 | 3.1 | 0.48 | 0.048 | 5 |
| | Medium-dose | 30 | 3.00 | 3.1 | 0.97 | 0.097 | 10 |
| | High-dose | 30 | 6.00 | 3.1 | 1.94 | 0.194 | 20 |

2.5 Administration of test product

The corresponding drug solution was administered via gavage using a syringe at a volume of 10 ml/kg. The model group and negative control group were administered with an equivalent volume of sterile injectable water. The administration dose for each animal was adjusted weekly according to the animal's latest weighed weight.

2.6 Determination of model mice

2.6.1 The tail suspension test was carried out to observe straightening reflex of limbs. When the mice were suspended by tail, normal mice exhibited natural straightening of bilateral hindlimbs, and mice affected by motor neuron disease exhibited flexion of hindlimbs. The scoring criteria are as follows:

0 points-No flexion of the hindlimbs observed.

1 point-Flexion observed in unilateral hindlimb, with no obvious flexion in the other.

2 points-Flexion observed in bilateral hindlimbs. Mice with a score of 2 points were considered to be in the symptomatic phase. The tail suspension test was stopped when all ALS mice reached a score of 2 points.

2.6.2 Hanging Test: The mouse was placed on a 3-mesh screen. The screen was then rapidly inverted, causing the mouse to hang upside down, and the timer was started. A 90 second cut-off time was set. The mice with a hanging time of less than 90 seconds were noted, and the actual time they remained hanged was recorded. The hanging test was discontinued upon the occurrence of death in ALS mice. ALS mice exhibited progressive motor function loss, manifested as flexion of hind limbs when suspended by the tail. The severity of this abnormality correlates with the degree of muscle atrophy and loss of muscle strength. In this study, the onset of disease in transgenic mice was determined based on the results of the tail suspension test. Additionally, guided by literature review and previous experimental experience, each mouse was sequentially evaluated by both the tail suspension and hanging tests.

2.7 Indicators, time and contents of observation

2.7.1 General observation and evaluation methods

During the test, animals were observed for general conditions including mental status, disease progression, and survival status. For each animal, the following data were recorded: time of birth, start time of dosing, body weight, and time of death. The mean survival time of animals and the cumulative death rate of animals at various time points were calculated.

Identification of endpoint of death: From an ethical standpoint, death of a mouse was determined according to the method reported in reference[4], namely if a mouse was unable to right itself from a lateral position on a flat surface to recover a normal posture within 25 seconds, it was considered dead.

2.7.2 Behavioral indicators

Neurological function was evaluated during the dosing phase using the Snow scoring method twice weekly. The specific criteria for neurological system scoring are as follows.

1.0 point--Abnormal extension or tremors of hind limbs of the mouse when suspended by tail.

1.5 points--Weakness of unilateral hind limb.

2.0 points--Weakness of bilateral hind limbs along with abnormal gait.

2.5 points--Obvious weakness of unilateral hind limb.

3.0 points--Obvious weakness of bilateral hind limbs.

3.5 points--Paralysis in unilateral hind limb.

4.0 points--Paralysis in bilateral hind limbs.

5.0 points--Inability of mouse to right itself from a supine position within 20 seconds.

The time from administration to the first occurrence of a neurological function score $\geq 3.5$ was defined as the time to onset of paralysis. The mean time to onset of paralysis of animals was calculated. The percentage of animals exhibiting paralysis at various time points after administration was calculated. The paralysis delay rate for each drug group was also calculated according to the following formula:

$$\% \text{ delay rate} = \frac{\text{time to onset of paralysis of administration group} - \text{time to onset of paralysis of model group}}{\text{time to onset of paralysis of model group}} \times 100\%.$$

2.7.3 Mouse exclusion criteria

During the test, the general health status and motor function changes of the mice were observed. The death that occurred outside the normal onset period or did not meet the progressive motor function decline pattern of ALS was considered as non-ALS death. No signs of disease were observed according to the criteria for ALS model. The measurement data of such mice were excluded from the final data statistical analysis.

2.8 Notification of relevant staff

The animal department should be notified when animals were purchased. The abnormal event of animals should be reported to the pathology department for processing.

2.9 Statistical methods

The experimental data were presented as mean $\pm$ standard deviation ($\bar{x} \pm s$). The statistical analysis was performed using statistical software SPSS 25.0. Survival analysis was performed using the Log Rank and Tarone-Ware tests. Survival curves were plotted using Graphpad prism 8.0. Indicators including the time to onset of paralysis and behavioral scores were firstly subjected to a normality test. For data that followed a normal distribution, means were compared by one-way ANOVA. If the variances were homogeneous, pairwise comparisons were performed using the least significant difference method. If the variances were not homogeneous, Dunnett's T3 test was used. For data that did not follow a normal distribution, non-parametric tests were used for statistical analysis.

2.10 Instrument system

JJ2000 electronic balance from Changshu Shuangjie Testing Instrument Factory

Sartorius BSA2201 electronic balance from Sartorius Scientific Instruments (Beijing) Co. Ltd.

Sartorius BT224S precision analytical balance from Sartorius Scientific Instruments (Beijing) Co. Ltd.

3. Results

3.1 Effect on survival time

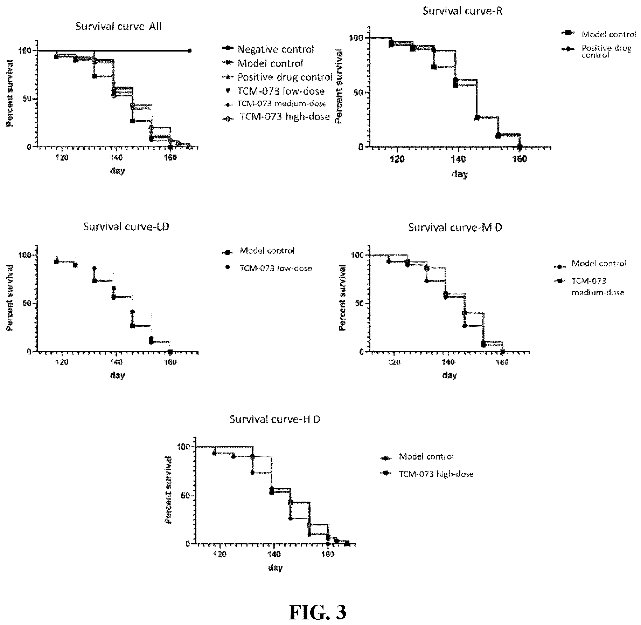

As shown in Table 4 and FIG. 3, there was no statistically significant difference in survival time between each administration group and the model group (P > 0.05). However, the mean survival time (days) of each administration group was slightly prolonged. Further analysis of survival time of animals of different genders (see Table 4 for details) show that female mice in the low- and high-dose groups exhibited a significantly prolonged mean survival time compared to those in the model group, and no significant difference in mean survival time was observed between the male mice of the treatment groups and the model group.

Table 4 Survival time

| Group | No division by gender | | Male | | Female | |
|---|---|---|---|---|---|---|
| | N | Survival time (day) | N | Survival time (day) | N | Survival time (day) |
| Model control group | 30 | 138.9±10.9 | 15 | 137.3±11.9 | 15 | 140.6±9.9 |
| Positive drug control group (Riluzole) | 26 | 141.4±9.9 | 12 | 140.4±13.0 | 14 | 142.3±6.6 |
| TCM-073 Low dose | 29 | 143.0±10.1 | 15 | 138.8±10.8 | 14 | 147.6±7.1* |
| Medium dose | 30 | 142.6±9.2 | 15 | 139.3±10.1 | 15 | 145.8±7.2 |
| High dose | 29 | 143.6±10.2 | 15 | 137.3±5.7 | 15 | 149.9±10.0* |

Note: * indicates $P$ <0.05 compared to the model group.

3.2 Effect on time to onset of paralysis

The time from administration to the first occurrence of a neurological function score ≥3.5 was defined as the time to onset of paralysis. The mean time to onset of paralysis of animals was calculated. For animals that died without reaching a paralysis score, the date of death was taken as the onset time of paralysis to ensure the number of animals was qualified for analysis. The results are shown in Table 5. The positive drug control group and all administration groups significantly delayed the time to onset of paralysis (P < 0.05). Further analysis of the time to onset of paralysis of animals of different gender show that for both female and male mice, the medium- and high-dose groups significantly delayed the onset of paralysis.

Table 5 Time to onset of paralysis

| Group | | No division by gender | | Male | | Female | |
|---|---|---|---|---|---|---|---|
| | | N | Time to onset of paralysis (day) | N | Time to onset of paralysis (day) | N | Time to onset of paralysis (day) |
| Model control group | | 30 | 129.5±12.6 | 15 | 122.5±12.7 | 15 | 136.5±7.9 |
| Positive drug control group (Riluzole) | | 26 | 134.8±14.2* | 12 | 129.2±18.9 | 14 | 139.6±5.7 |
| TCM-073 | Low dose | 29 | 136.9±10.5* | 15 | 132.1±11.5 | 14 | 142.1±6.0 |
| | Medium dose | 30 | 137.7±11.1** | 15 | 132.9±12.7* | 15 | 142.6±6.7* |
| | High dose | 30 | 138.4±8.4* | 15 | 132.7±4.4* | 15 | 144.2±7.6* |

Note: * and ** indicate $P$ <0.05 and $P$ <0.01 compared to the model group, respectively.

3.3 Effects on hanging time and neurological function score

As shown in Tables 6 and 7, both the medium-dose group (on D39 and D46 of administration) and the high-dose group (on D31 and D34 of administration) significantly reduced the impairment degree of neurological functions compared to the model group (P < 0.05). The high-dose group and the positive drug control group significantly prolonged the hanging time on D46 of administration compared to the model group (P < 0.05).

Table 6 Neurological function score

| Group | | N | Neurological function score | | | | |
|---|---|---|---|---|---|---|---|
| | | | 31d | 34d | 39d | 42d | 46d |
| Negative control group | | 20 | 0±0 | 0±0 | 0±0 | 0±0 | 0±0 |
| Model control group | | 30 | 2.2±0.4 | 2.2±0.5 | 2.3±0.5 | 2.3±0.5 | 2.4±0.6 |
| Positive drug control group (Riluzole) | | 26 | 2.2±0.4 | 2.2±0.4 | 2.2±0.5 | 2.3±0.5 | 2.2±0.4 |
| TCM-073 | Low dose | 29 | 2.1±0.2 | 2.1±0.3 | 2.2±0.4 | 2.2±0.3 | 2.2±0.3 |
| | Medium dose | 30 | 2.1±0.4 | 2.1±0.4 | 2.1±0.4* | 2.2±0.4 | 2.2±0.4* |
| | High dose | 30 | 2.0±0.1* | 2.0±0.1* | 2.1±0.2 | 2.1±0.3 | 2.2±0.3 |

Note: * indicates $P$ <0.05 compared to the model group.

Table 7 Hanging time

| Group | | N | Hanging time (s) | | | | |
|---|---|---|---|---|---|---|---|
| | | | 31d | 34d | 39d | 42d | 46d |
| Negative control group | | 20 | 90.0±0 | 90.0±0 | 90.0±0 | 90.0±0 | 90.0±0 |
| Model control group | | 30 | 49.5±31.4 | 45.4±32.0 | 40.4±33.3 | 30.8±30.7 | 20.0±22.3 |
| Positive drug control group (Riluzole) | | 26 | 63.3±29.2 | 57.6±32.6 | 46.5±29.8 | 40.8±30.7 | 33.8±27.2* |
| TCM-073 | Low dose | 29 | 57.1±29.7 | 48.7±24.3 | 40.3±19.5 | 34.4±24.2 | 24.7±19.9 |
| | Medium dose | 30 | 57.6±30.7 | 53.7±29.6 | 45.4±30.2 | 35.1±29.5 | 28.2±29.2 |
| | High dose | 30 | 49.9±30.0 | 52.7±27.8 | 47.8±22.8 | 39.2±24.9 | 30.7±22.2* |

Note: * indicates $P$ <0.05 compared to the model group.

4. Conclusion

SOD1-G93A transgenic mice, a classic model of ALS, were used for the test. The mice started to be administered and observed from 70 days of age, and the therapeutic effects of various doses of the pharmaceutical composition of the present invention were evaluated. The results show that each dose group of the test drug can delay the onset of paralysis. The medium- and high-dose groups (3.0 g and 6.0 g raw drug/kg, respectively) can improve the impaired neurological function of SOD1-G93A transgenic mice to varying degrees, and the high-dose group (6.0 g raw drug/kg) can prolong the hanging time of the mice. No significant therapeutic effect was observed in each administration group in terms of prolonging survival time.

5 Record and storage

Except for data collected directly by computers or automated instruments, all other data obtained in actual research were recorded on forms or record sheets and organized and bound in time. All recorded data were signed by the operator and verifier, and the date of recording was noted. When the final report was submitted, all original information, documents, and other relevant materials were submitted to the archive room for storage.

Experimental example 3: Effect of the pharmaceutical composition of the present invention on disuse muscle atrophy of skeletal muscles of mice with hindlimb suspended

Experimental objective

The muscle atrophy model of mice was established by suspending hindlimbs of mice to mimic clinical sarcopenia, and whether the pharmaceutical composition of the present invention has a protective and therapeutic effect on skeletal muscle atrophy was observed.

1. Experimental materials

1.1 Test product: The pharmaceutical composition of the present invention was prepared as in Example 2, which was abbreviated as TMC-073.

1.2 Positive control product and main reagents

1.2.1 Positive control product: Whey protein powder was manufactured by American Fierte Pharmaceutical Group Co., Ltd. and stored in a cool and dry place, away from direct sunlight. Test product number: GLPTN-2106-D2. Batch number: 03172023B. Expiration date: March 17, 2023.

1.2.2 Main reagents:

Sterile water for injection from Shijiazhuang No. 4 Pharmaceutical Co., Ltd. Batch number: 2009053201. Expiration date: 2022.09.04.
10% neutral buffered formalin as a fixative from Beijing Yili Fine Chemicals Co., Ltd. Batch number: 20220406. Expiration date: 2023.04.05.
Isoflurane was purchased from Beijing Youcheng Jiaye Biotechnology Co., Ltd. Batch number: G45780. Expiration date: 2024.12.30.

1.3 Experimental system

1.3.1 Animal Strain: C57BL/6J.
1.3.2 Animal Grade: SPF grade.
1.3.3 Animal Gender and Quantity: A total of 100 animals were purchased, with an equal number of males and females. 96 animals were actually used in the test.
1.3.4 Age of animals at the start of dosing: The protocol required animals to be 5-7 weeks old, and the animals actually used were 5-7 weeks old.
1.3.5 Weight of animals at the start of dosing: The protocol required 22-24 g for male mice and 21-23 g for female mice, and the weight of mice actually used was 20.7-24.6 g for male mice and 16.6-19.5 g for female mice.
1.3.6 Animal Source: Beijing Vital River Laboratory Animal Technology Co., Ltd.
1.3.7 Animal Certification of Quality, issuing authority, and receipt date: Certification numbers: 110011220106187852 and 110011220106187965. License number SCXK (Beijing) 2021-0006. Issuing authority: Beijing Vital River Laboratory Animal Technology Co., Ltd. Receipt date: June 22, 2022.
1.3.8 Feeding Conditions: Mice were reared in cages at the Innovative Drug Evaluation Center of Hebei Research Institute of Integrated Traditional Chinese and Western Medicine. License Number: SYXK (Hebei) 2020-006. Lighting: 12 h/day. Temperature: 20-26°C. Relative Humidity: 40%-70%.
1.3.9 Quarantine process: Animals were subjected to quarantine for 7 days, during which no abnormalities were observed in their water and food intake and health status.

1.3.10 Feed: Rat and mice maintenance feed (SPF-grade) was provided by Beijing Keao Xieli Feed Co., Ltd. Feed Production License Number: Beijing Feed Certificate (2018) 06073. Certificate Number: 1112622200083240. Batch Number: 22043213.

1.3.11 Bedding: Corn cob bedding (common grade) was provided by Beijing Keao Xieli Feed Co., Ltd. The bedding was sterilized by high temperature and high pressure before use. Batch Number: 22049811.

1.3.12 Water: Clean water for drinking was prepared using the ROB-B drinking water system for laboratory animal, and filled in water bottles for free drinking.

1.3.13 Marking: Mice were marked with ear tags, provided by Globalebio (Beijing) Technology Co., Ltd.

## 2 Test methods

### 2.1 Test design basis

2.1.1 Adopted standards were determined according to the Registration, Classification and Submission Data Requirements for Chinese Traditional Medicine issued by the State Food and Drug Administration, Good Laboratory Practice for non-clinical laboratory studies, and related literatures.

2.1.2 Description of selected experimental system: Skeletal muscle possesses rapid adaptability to external environmental changes. In response to unloading conditions such as reduced physical activity, immobilization, prolonged bed rest, or weightlessness, skeletal muscle exhibits decreased muscle mass and/or muscle strength, or impaired muscular physiological function. The hindlimbs of mice were suspended to cause disuse muscle atrophy, which exhibited obvious characteristics of sarcopenia, for mimicking the human sarcopenia and studying the prevention and treatment of human sarcopenia, and it is a useful alternative method.

2.1.3 Description of selected positive control product: Currently, therapies for sarcopenia primarily include drug treatments (e.g., testosterone, growth hormone), protein supplementation, and resistance training. However, supplementing drugs such as testosterone and growth hormone may lead to adverse effects including fluid retention, increased risk of prostate cancer, and erythrocytosis, due to which they lack wide spread application in clinical. Currently, the nutritional protein supplementation and resistance training are commonly used symptomatic treatments in clinical. Therefore, in this test, whey protein powder was selected as the positive control product. Studies have shown that an adult dose of whey protein ranging from 0.5 to 1 g/kg/day can improve muscle strength and function in patients with sarcopenia. Accordingly, a daily dose of 10 g/kg was applied to mice, which corresponds to 10 times the clinical dose.

### 2.2 Dosage and grouping

Based on body weight, 96 mice were selected from 100 mice and randomly grouped into blank control group, model control group, positive control group (whey protein powder), TCM-073 low-dose group (1.50 g raw drug/kg), TCM-073 medium-dose group (3.00 g raw drug/kg), and TCM-073 high-dose group (6.00 g raw drug/kg). Each group included 16 animals, with an equal number of males and females. The specific grouping and dose settings are shown in Table 8.

Table 8 Grouping and dose settings

| Group | | Number of animals | Dose (g raw drug/kg) | Specification (g raw drug /g powder) | Dose for formulation (g powder/kg) | Formulation concentration (g powder/ml) | Multiple relative to clinical equivalent dose |
|---|---|---|---|---|---|---|---|
| blank control group | | 16 | - | - | - | - | - |
| model control group | | 16 | - | - | - | - | - |
| positive control group | | 16 | 10 | - | - | 1 | 10 |
| TCM-073 | Low dose | 16 | 1.50 | 3.1 | 0.48 | 0.048 | 5 |
| | Medium dose | 16 | 3.00 | 3.1 | 0.97 | 0.097 | 10 |
| | High dose | 16 | 6.00 | 3.1 | 1.94 | 0.194 | 20 |

2.3 Model Establishment

Except for the animals in the blank control group, the tails of mice in all other groups were fixed with a fixing device to the top of the cage at an angle of less than 30° between the mouse trunk and the ground, allowing free 360° rotation and movement. A model of disuse muscle atrophy of hindlimb skeletal muscle was thus established by lifting the hindlimbs of mice off the ground to remove weight-bearing, so as to simulate clinical sarcopenia.

During the suspension of hindlimbs, one mouse per cage was suspended for 14 days. Then the suspension was removed and the hind limbs were released to self-recover for 9 days.

2.4 Administration method

The drug was orally administered via gavage, consistent with the clinically recommended oral administration.

2.5 Preparation and storage of test product

The proposed clinical dose of TCM-073 was 18 g of raw drug/day. In this test, three doses were set, namely low dose (1.5 g raw drug/kg), medium dose (3.0 g raw drug/kg) and high dose (6.0 g raw drug/kg), corresponding to 5, 10, and 20 times the clinical dose, respectively (See Table 1 for details). An appropriate amount of the drug was weighed, ground and mixed evenly with sterile injectable water as the solvent, and the mixture was adjusted to the corresponding volumes to prepare high, medium, and low doses. The three doses were prepared just before use.

2.6 Administration of test product

The corresponding drug solution was administered at a dosing volume of 10 ml/kg via gavage using a syringe. The model control group and blank control group were administered with an equivalent volume of sterile injectable water. The administration was started on the day of starting hindlimb suspension and continued for 23 days.

2.7 Indicators, time and contents of observation

2.7.1 General observation

During the suspension of hindlimbs, it was ensured that animals under suspension status were able to access food and water using only their forelimbs. Animals were observed for any signs of distress or pain.

Body weights of animals were recorded on day 1 and day 14 of suspension, on day 7 of the self-recovery period, and on the day of necropsy.

2.7.2 Measurement of behavioral indicators

The grip strength was measured on day 13 of suspension of hindlimbs and day 6 of the self-recovery period.

A grip strength meter was placed horizontally so that the grip grid moved horizontally. For each mouse, grip strength of hindlimbs was measured three times. The average value of the three readings was used to assess the effect of the test drug on muscle strength of limbs of animals.

2.7.3 Measurement of muscle-related indicators

2.7.3.1 Measurement of blood flow of hindlimb muscle by Laser Doppler Imaging

On the day after the final administration, mice were anesthetized and placed in a prone position. The skin of the left hindlimb was cut to expose a prominent vessel on the surface of gastrocnemius muscle (small saphenous vein). Blood flow in the vessel over a 2-minute period was recorded.

2.7.3.2 Wet weight of muscle

After the measurement of blood flow of hind limbs was completed, mice were sacrificed by cervical dislocation. The gastrocnemius and soleus muscles of mice were harvested from the left hindlimb and the moisture on the surface was removed with filter papers. These tissues were weighed and the muscle index was calculated according to the following formula:

$$\text{Muscle index} = (\text{Muscle weight (g)} / \text{body weight (g)}) \times 1000$$

2.7.3.3 Histopathological examination

The gastrocnemius and soleus muscles were harvested from the right hindlimb, fixed in 10% neutral buffered formalin, and subjected to HE staining, and the histological changes of muscles (gastrocnemius and soleus muscles) were observed.

2.8 Notification of relevant staff

The animal department should be notified when animals were purchased. The abnormal event of animals should be reported to the pathology department for processing.

2.9 Instrument system

Sartorius BSA2201 electronic balance, Sartorius (Shanghai) Trading Co., Ltd.
Sartorius BT224S precision analytical balance, Sartorius (Shanghai) Trading Co., Ltd.
NVT2201ZH electronic balance, OHAUS Instrument (Shanghai) Co., Ltd.
BlueSpin standard magnetic stirrer, Dalong Xingchuang Lab Equipment (Beijing) Co., Ltd.
DEF2-002 grip strength gauge, Chatillon Force Measurement.
MoorLDI2-HIR Laser Doppler Imaging System, Moor Instruments Ltd.
R500IE Small Animal Anesthesia Machine, Shenzhen RWD Life Technology Co., Ltd.

2.10 Statistical methods

The experimental data were presented as mean $\pm$ standard deviation ($\overline{x}\pm s$). The statistical analysis was performed using statistical software SPSS 26.0. A normality test was performed firstly. For data that followed a normal distribution, means were compared by one-way ANOVA. If the variances were homogeneous, pairwise comparisons were performed using the least significant difference method. If the variances were not homogeneous, Dunnett's T3 test was used. For data that did not follow a normal distribution, non-parametric tests were used for statistical analysis.

3. Results

3.1 Effect on body weight

As shown in Table 9, during the test, there was no significant difference in body weight between the mice of the model control group mice and the blank control group. There was no significant difference in the body weight of mice in the positive control group and each TCM-073 dose group compared to the model control group.

Table 9 Effect of TCM-073 on body weight of mice (g) with disuse muscle atrophy caused by hindlimbs suspension

| Group | N | D1 | D14 | D21 | D24 |
|---|---|---|---|---|---|
| Blank control group | 16 | 19.6±2.2 | 21.0±2.0 | 21.7±2.1 | 22.0±2.0 |
| Model control group | 16 | 20.1±2.6 | 21.2±2.3 | 21.4±2.5 | 22.1±2.6 |
| Positive control group | 16 | 20.4±2.4 | 21.1±1.9 | 21.2±2.1 | 21.4±2.2 |
| TCM-073 low dose | 16 | 20.0±2.3 | 20.9±2.1 | 20.8±2.3 | 21.4±2.3 |
| TCM-073 medium dose | 16 | 20.1±2.1 | 20.9±2.0 | 20.9±2.2 | 21.2±2.3 |
| TCM-073 high dose | 16 | 20.2±2.4 | 21.2±2.0 | 21.4±2.5 | 21.8±2.6 |

3.2 Effects on grip strength of hindlimbs

As shown in Table 10, on day 13 of suspension of hindlimbs (day 13 of administration), the grip strength of mice in each group was measured. The results show that the grip strength of hindlimbs of mice in the model control group was significantly reduced compared to the blank control group ($p<0.01$). The grip strength of hindlimbs of mice in the other administration groups showed varying degrees of increase compared to the model control group, wherein the positive control group and the TCM-073 high-dose group showed significant differences ($p<0.05$, $p<0.01$). On day 6 of self-recovery (day 20 of administration), the results of grip strength test show that the grip strength of hindlimbs of mice in the model control group was still significantly lower than that in the blank control group (p<0.05). Compared to the model control group, the grip strength of mice in the TCM-073 high-dose group was significantly increased (p<0.05), and the grip strength of mice in the other administration groups also showed varying degrees of increase, but no significant differences were observed.

Table 10 Effect of TCM-073 on grip strength of hindlimbs of mice (N) with disuse muscle atrophy caused by suspension of hindlimbs

| Group | N | D13 | D20 |
|---|---|---|---|
| Blank control group | 16 | $1.78 \pm 0.21$ | $1.95 \pm 0.24$ |
| Model control group | 16 | $1.51 \pm 0.15^{\triangle\triangle}$ | $1.77 \pm 0.25^{\triangle}$ |
| Positive control group | 16 | $1.63 \pm 0.20^{*}$ | $1.89 \pm 0.21$ |
| TCM-073 low dose | 16 | $1.58 \pm 0.14$ | $1.89 \pm 0.18$ |
| TCM-073 medium dose | 16 | $1.56 \pm 0.12$ | $1.79 \pm 0.19$ |
| TCM-073 high dose | 16 | $1.71 \pm 0.14^{**}$ | $1.94 \pm 0.21^{*}$ |

Note: $\triangle$ and $\triangle\triangle$ indicate p<0.05 and p<0.01 compared to the blank control group, respectively. * and ** indicate p<0.05 and p<0.01 compared to the model control group, respectively.

3.3 Effects on blood flow in the hindlimb muscles

As shown in Table 11, the venous blood flow of the hindlimbs of mice in the model control group was significantly reduced compared to the blank control group (p<0.05). The blood flow of the hindlimbs of mice in the positive control group was significantly increased compared to the model control group (p<0.05). Blood flow of hindlimbs of mice in each TCM-073 dose group also exhibited varying degrees of increase, with the high-dose group showing the most significant increase (p<0.01).

Table 11 Effect of TCM-073 on blood flow of the left hindlimb of mice with disuse muscle atrophy caused by suspension of hindlimbs

| Group | N | Flux |
|---|---|---|
| Blank control group | 16 | $921.5 \pm 471.7$ |
| Model control group | 16 | $545.2 \pm 368.0^{\triangle}$ |
| Positive control group | 16 | $833.0 \pm 256.3^{*}$ |
| TCM-073 low dose | 16 | $682.4 \pm 471.2$ |
| TCM-073 medium dose | 16 | $676.6 \pm 280.1$ |
| TCM-073 high dose | 16 | $922.3 \pm 325.4^{**}$ |

Note: $\triangle$ indicates p<0.05 compared to the blank control group. * and ** indicate p<0.05 and p<0.01 compared to the model control group, respectively.

3.4 Effects on muscle weight of hindlimbs

The wet weights of the gastrocnemius and soleus muscles of mice were converted to the corresponding muscle mass indexes through body weight correction. As shown in Table 12, compared to the blank control group, the model control group showed a significantly lower gastrocnemius muscle mass index (p<0.05), and the soleus muscle mass index was lower but not significantly different. The gastrocnemius muscle mass index and soleus muscle mass index of mice in the positive control group and each TCM-073 dose group showed varying degrees of increase compared to the model control group, but no significant differences were observed.

Table 2 Effect of TCM-073 on muscle mass index of hindlimbs of mice (mg/g) with disuse muscle atrophy caused by suspension of hindlimbs

| Group | N | Gastrocnemius muscle mass index | Soleus muscle mass index |
|---|---|---|---|
| Blank control group | 16 | $5.0702 \pm 0.6604$ | $0.9728 \pm 0.4392$ |
| Model control group | 16 | $4.4267 \pm 0.8391^{\triangle}$ | $0.8351 \pm 0.3795$ |

(continued)

| Group | N | Gastrocnemius muscle mass index | Soleus muscle mass index |
|---|---|---|---|
| Positive control group | 16 | 4.9233±0.7255 | 0.9162±0.3089 |
| TCM-073 low dose | 16 | 4.8221±0.9643 | 1.0909±0.5874 |
| TCM-073 medium dose | 16 | 4.6607±0.7519 | 0.9695±0.4405 |
| TCM-073 high dose | 16 | 4.7012±0.8769 | 0.9825±0.5694 |

Note: $\triangle$ indicates $p<0.05$ compared to the blank control group.

3.5 Effects on histomorphology of hindlimb muscle

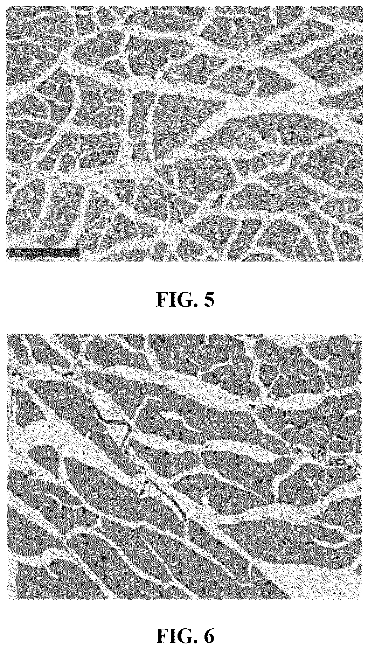

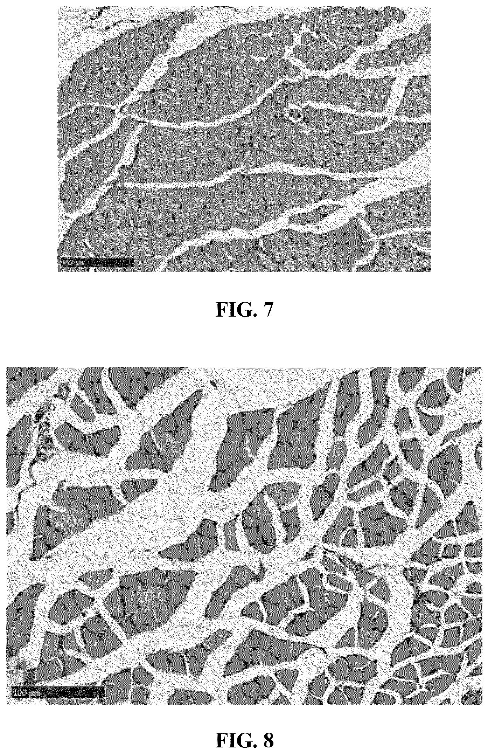

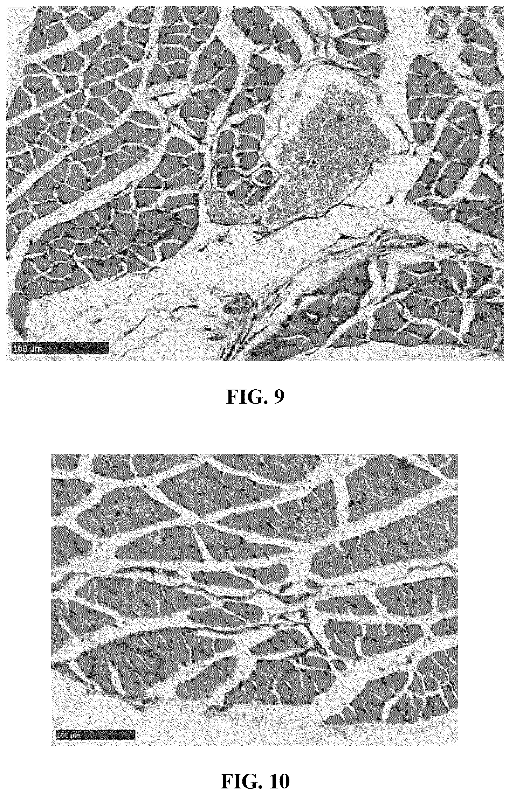

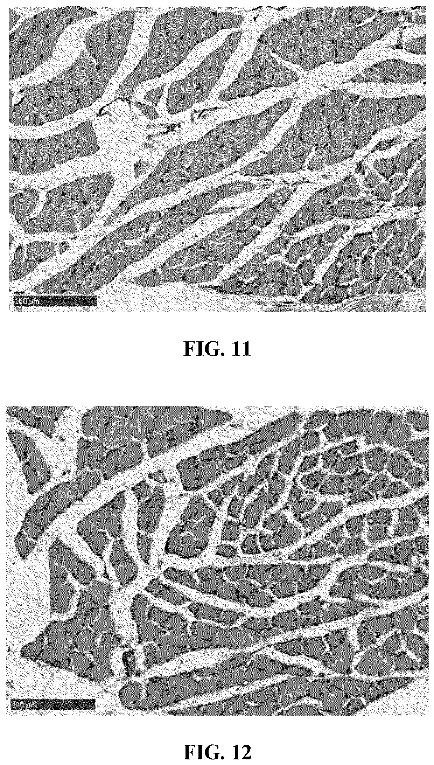

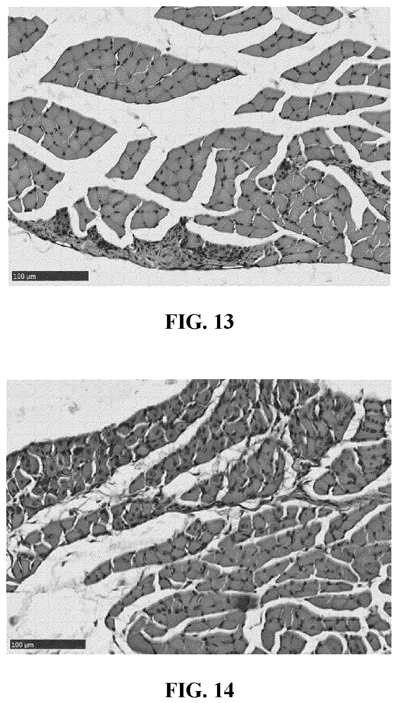

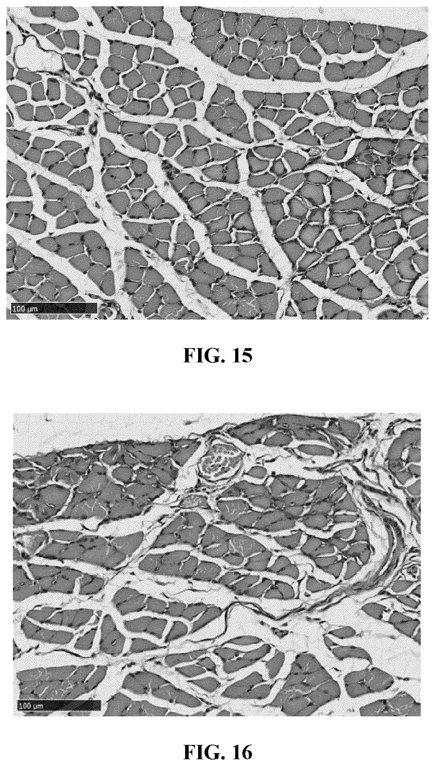

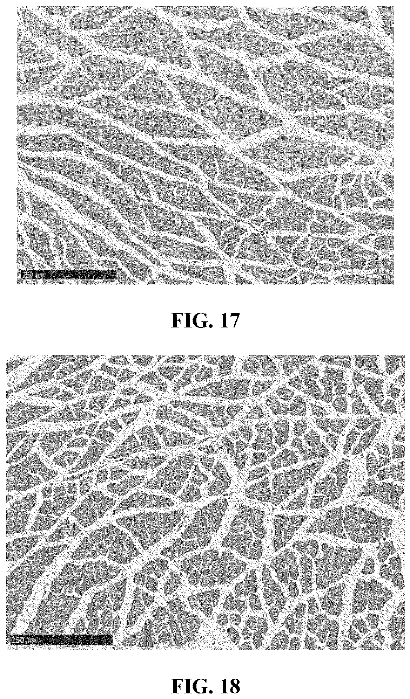

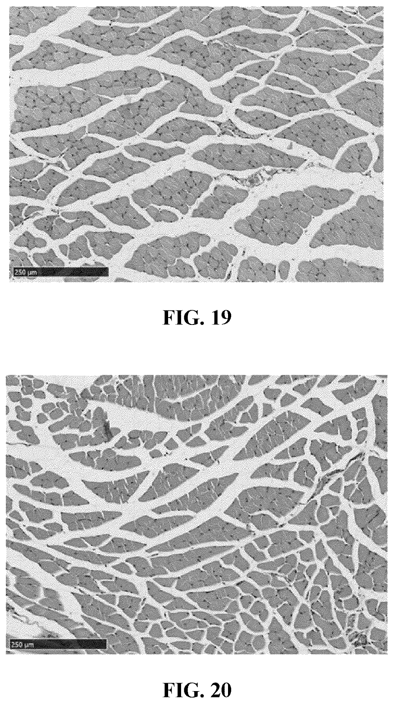

3.5.1 The results of pathological examination in this test show that the soleus muscle in the model control group exhibited mild muscle atrophy (microscopically characterized by reduced muscle fiber volume and presence of angular muscle fibers) compared to the blank control group. Compared to the model control group, no significant improvement in soleus muscle lesions was observed in each administration group. No significant lesions were observed in the gastrocnemius muscle of either the model control group or all administration groups, as shown in FIGs. 5-20.

3.5.2 Effects on cross-sectional area of muscle fiber

The histomorphology examination of muscle shows that the soleus muscle exhibited greater atrophy in response to suspension of mice hindlimbs than the gastrocinus muscle. The cross-sectional area of soleus muscle fibers of mice from each group was measured using the digital pathological sections viewing software NDP.view2 (U12388-01). As shown in Table 13, the cross-sectional area of soleus muscle fibers of mice in the model control group was significantly reduced compared to the blank control group ($p<0.01$). Compared to the model control group, the cross-sectional area of the soleus muscle fibers of mice in the positive control group and the TCM-073 high-dose group was significantly increased ($p<0.01$), and the cross-sectional area of the soleus muscle fibers of mice in the TCM-073 low- and medium-dose groups was also increased, but not significantly different.

Table 13 Effect of TCM-073 on cross-sectional area ($\mu m^2$) of soleus muscle fiber of mice with disuse muscle atrophy caused by suspension of hindlimbs

| Group | N | Cross-sectional area |
|---|---|---|
| Blank control group | 11 | 589.6±158.5 |
| Model control group | 12 | 333.8±28.5$^{\triangle\triangle}$ |
| Positive control group | 14 | 401.5±61.9** |
| TCM-073 low dose | 13 | 352.0±109.0 |
| TCM-073 medium dose | 12 | 357.8±50.3 |
| TCM-073 high dose | 11 | 410.8±75.8** |

Note: $\triangle\triangle$ indicates $p<0.01$ compared to the blank control group. ** indicates $p<0.01$ compared to the model control group.

4. Conclusion

A sarcopenia model was successfully established by suspending the hindlimbs of mice for 14 days. Under the conditions of this test, the pharmaceutical composition of the present invention can significantly ameliorate the disuse muscle atrophy of mice caused by suspension of hindlimbs, and has the effects of increasing muscle strength and muscle fiber fullness and increasing blood supply to muscle tissue.

5. Discussion

Sarcopenia is a syndrome characterized by decline in muscle function and mass due to atrophy and loss of muscle fibers. Muscle fiber atrophy refers to reduction in muscle volume caused by nutritional disturbance of striated muscle and muscle fiber becoming thinner or even disappearing, which is mostly caused by muscle disorders or neurological system dysfunction. Major etiologies include neurogenic muscle atrophy, myogenic muscle atrophy, disuse muscle atrophy, and muscle atrophy due to other causes. Muscle mass is maintained by the dynamic balance between continuous degradation and synthesis of skeletal muscle. The persistently reduced mechanical loading on skeletal muscle may reduce the mass and function of skeletal muscle, resulting in disuse muscle atrophy. The most direct change in morphology and structure of muscles under disuse is muscle fiber atrophy, primarily manifested as reduced muscle mass, decreased muscle volume, decreased cross-sectional area of muscle fibers, and change in muscle fiber type. Changes in functions of muscles under disuse primarily include reduced muscle strength, and changes in neuromuscular function and ability of fatigue resistance.

In this test, the weight-bearing on hindlimbs was removed by suspending hindlimbs of mice, leading to changes in muscle function and significant reduction in grip strength of hindlimbs. Moreover, the morphology and structure of muscles was significantly changed, including decrease in mass of skeletal muscle of hindlimbs, muscle fiber atrophy, and reduced cross-sectional area of muscle fibers. This test successfully mimicked the clinical characteristics of human sarcopenia.

In the development of disuse muscle atrophy, different muscles exhibit varying rates of muscle fiber loss. The soleus muscle shows greater rate and extent of muscle fiber loss compared to the gastrocnemius. As observed in this test, after the mice self-recovered from suspension of hindlimbs, the histomorphological examination showed that the gastrocnemius muscle had largely returned to normal, with no obvious pathological changes compared to the blank control group. However, the soleus muscle still exhibited muscle fiber atrophy. The cross-sectional area of soleus muscle fibers of mice from each group was measured and compared. The results showed that the cross-sectional area of soleus muscle fibers of mice in each TCM-073 dose group was larger than that of the model control group, wherein the most pronounced difference was observed in the TCM-073 high-dose group (6.00 g raw drug /kg). In addition, the grip strength test of mice was carried out. The results showed that mice in the TCM-073 high-dose group (6.00 g raw drug/kg) exhibited significantly greater grip strength of hindlimbs during both the suspension of hindlimbs and self-recovery period compared to the model control group, indicating that TCM-073 can promote the recovery of grip strength of mice with muscle atrophy. Since muscle strength is related to the cross-sectional area of muscle fibers and fiber number, the improvement in grip strength of hindlimbs of mice with disuse atrophy by TCM-073 may be associated with this fact.

Capillaries play a crucial role in delivering oxygen and nutrients to skeletal muscle. The capillary network of skeletal muscle changes in response to physiological or pathological conditions. The long-term inactivity not only leads to a reduction in skeletal muscle fibers but also induces regression of capillary of skeletal muscle. The small saphenous veins are prominent vessels on the surface of the gastrocnemius muscle, responsible for collecting venous blood from the superficial compartment of the lower leg. The superficial posterior muscle group of lower legs includes the gastrocnemius, soleus, and plantaris. In this test, a laser Doppler imager was used to monitor blood flow of the small saphenous vein. Results show that the high dose of TCM-073 (6.00 g raw drugt/kg) can significantly increase blood supply to the lower leg skeletal muscles of mice with disuse muscle atrophy.

Therefore, the pharmaceutical composition of the present invention exhibits certain protective and therapeutic effects against decrease in muscle strength and muscle atrophy of sarcopenia, potentially by increasing blood supply to muscle and muscle fiber fullness.

6. Additional notes

At the time of receipt, female mice actually weighting 17-19 g were received to ensure that all experimental mice were within the same age range in week. Due to the naturally slow growth rate of female mice, their body weight at the start of dosing did not reach the weight range required by the protocol. However, as body weight was deemed to have no significant impact on the experimental outcomes, the experiment was conducted as planned.

7. Record and storage

Except for data collected directly by computers or automated instruments, all other data obtained in actual research were recorded on forms or record sheets and organized and bound in time. All recorded data were signed by the operator and verifier, and the date of recording was noted. When the final report was submitted, all original information, documents, and other relevant materials were submitted to the archive room for storage.

7.1 Storage unit: Innovative Drug Evaluation Center of Hebei Yiling Pharmaceutical Research Institute

7.2 Address: No. 238, Tianshan Street, Shijiazhuang

Experimental example 4: Study on the action mechanism of the pharmaceutical composition of the present invention in treating SOD1-G93A transgenic mice with amyotrophic lateral sclerosis

Experimental objective

The action mechanism of pharmaceutical composition of the present invention in treating amyotrophic lateral sclerosis was explored using SOD1-G93A transgenic mice.

1. Experimental materials

1.1 Test product: The pharmaceutical composition of the present invention was prepared as in Example 2, which was abbreviated as TMC-073.

1.2 Positive drugs and main reagents

1.2.1 Positive drugs: Riluzole (brand name: Rilutek): 50 mg per tablet. Riluzole is indicated for the treatment of amyotrophic lateral sclerosis. It can extend survival period of patients and/or delay the need for tracheostomy. For adults, an oral administration of 50 mg (one tablet) twice daily should be adopted in clinical. It was manufactured by Sanofi Winthrop Industrie and packaged by Sanofi (Beijing) Pharmaceuticals Co., Ltd. Batch Number: 0KP4C. Expiration date: May 2023.
1.2.2 Main reagents: Sterile water for injection: Shijiazhuang No. 4 Pharmaceutical Co., Ltd. Batch Number: 2007023202. Expiration date: 2022.07.01.

1.3 Experimental system

1.3.1 Animal Strain: B6SJL-Tg (SOD1-G93A) 1Gur/J (Stock Number: 002726) provided by The Jackson Laboratory (United States). Maintaining and breeding strain, and identifying the offspring of SOD1-G93A transgenic mice were conducted by Jiangsu GemPharmatech Co., Ltd. Littermates that did not carry the mutant human SOD1 gene were used as the negative control.

1.3.2 Animal Grade: SPF grade.

1.3.3 Animal Gender and Quantity: Eighty B6SJL-Tg (SOD1-G93A) 1Gur/J mice. Twenty littermates that did not carry the mutant human SOD1 gene were used. Both males and females were included.

1.3.4 Animal Age: 7-8 weeks old.

1.3.5 Animal Weight: At the start of dosing, the body weight of male mice was 23.9-31.3 g, and the body weight of female mice was 17.7-25.4 g.
1.3.6 Animal Source: Purchased from The Jackson Laboratory (United States). Breeding was conducted by Jiangsu GemPharmatech Co., Ltd.
1.3.7 Animal Certification of Quality, issuing authority, and receipt date: Certification number 320727210100609023. License number SCXK (Jiangsu) 2018-0008. Issuing authority: Jiangsu GemPharmatech Co., Ltd. Receipt date: October 13, 2021.
1.3.8 Feeding Conditions: Mice were reared in cages at the Innovative Drug Evaluation Center of Hebei Research Institute of Integrated Traditional Chinese and Western Medicine. License Number: SYXK (Hebei) 2020-006. Lighting: 12 h/day. Temperature: 20-26°C. Relative Humidity: 40%-70%.
1.3.9 Quarantine Process: Animals were subjected to adaptive feeding for at least 5 days. During this period, whether the animals had normal water and food intake, and whether the animals were in good health status, had illness and showed signs of mortality were observed.
1.3.10 Feed: Rat and mice maintenance feed (SPF-grade) was provided by Beijing Keao Xieli Feed Co., Ltd. Feed Production License Number: Beijing Feed Certificate (2018) 06073. Experimental Animal Production License Number: SCXK (Beijing) 2019-0003. Product Batch Number: 21073213. Expiration date: 2022.01.03.
1.3.11 Water: Clean water prepared by the ROB-B drinking water system for laboratory animal was provided, and filled in water bottles for free drinking. The drinking water was detected annually for all indicators, including mainly microbiological indicators, toxicological indicators, sensory characteristics, and general chemical parameters, and the detection was commissioned to a qualified institution. Microbial limit detection was performed every 2 months, which was commissioned to the Quality Control Department of Shijiazhuang Yiling Pharmaceutical Co., Ltd. The detection reports were stored by the animal department and archived annually.
1.3.12 Bedding: Corn cob bedding (common grade) provided by Beijing Keao Xieli Feed Co., Ltd., was sterilized by high temperature and high pressure before use. Experimental Animal Production License Number: SCXK (Beijing) 2020-0010. Batch Number: 21099811.

1.3.13 Marking: Mice were marked with ear tags, provided by Globalebio (Beijing) Technology Co., Ltd.

2 Test methods

2.1 Test design basis

2.1.1 Adopted standards were determined according to the Registration, Classification and Submission Data Requirements for Chinese Traditional Medicine issued by the State Food and Drug Administration, Good Laboratory Practice for non-clinical laboratory studies, and related literatures.

2.1.2 Description of selected experimental system: Currently, the transgenic B6SJL-Tg(SOD1-G93A)1 Gur/J mice carrying a mutant human Cu/Zn superoxide dismutase (SOD) gene is considered the most commonly used and best animal models for amyotrophic lateral sclerosis (ALS). The pathogenesis of ALS remains largely unknown, with 90% of cases being sporadic and only 10% familial (FALS). In 1993, Rosen found missense mutations in the SOD1 gene, which was a significant milestone in ALS research. The identification of SOD1 mutations in FALS brought the subsequent development of animal models for ALS. Nowadays, the SOD1-G93A mice are widely used as the transgenic mice model for ALS both in clinical and in basic research, which carry high copies of mutant human SOD1 gene. Homozygous mice exhibit a reduction in spinal motor neurons and gradually exhibit neuromuscular dysfunction at around 3 months after birth, including limb tremor under tail suspension and hemiplegia of one or more limbs. Their lifespan typically is about 4-5 months. This model can effectively mimic the chronic progressive course of ALS along with some pathological and biochemical characteristics of the disease.

2.2 Dosage and grouping

After ALS model mice were determined to have significant changes compared to negative control mice in behavioral test by combining literature as well as prior experience, they were randomly grouped according to their body weight. The drug administration was started and mice were observed. The groups were specifically divided into model control group (M), positive drug control group (Riluzole, 0.02 g/kg, R), and TCM-073 low-dose (073-L), TCM-073 medium-dose (073-M), and TCM-073 high-dose groups (073-H),with 16 animals in each group. Additionally, wild-type littermates that did not carry mutant human SOD1 gene were used as the negative control group (C; a total of 20 mice). Specific experimental groupings and doses are shown in Table 14.

Table 14 Experimental grouping and dose setting

| Group | | Number of animals | Dose (g crude drug/kg) | Specification (g raw drug/g powder) | Dose for formulation (g powder/kg) | Formulation concentration (g powder/ml) | Multiple relative to clinical equivalent dose |
|---|---|---|---|---|---|---|---|
| Negative control group | | 20 | - | - | - | - | - |
| Model control group | | 16 | - | - | - | - | - |
| Positive drug control group (Riluzole) | | 16 | 0.02 g/kg | 50mg/tablet | - | 25ml/tablet | 10 |
| TCM-073 | Low dose | 16 | 1.50 | 3.1 | 0.48 | 0.048 | 5 |
| | Medium dose | 16 | 3.00 | 3.1 | 0.97 | 0.097 | 10 |
| | High dose | 16 | 6.00 | 3.1 | 1.94 | 0.194 | 20 |

2.3 Administration method

The drug was orally administered via gavage, consistent with the clinically recommended oral administration.

2.4 Preparation and storage of test product

The clinical dose of Riluzole was calculated as approximately 0.002 g/kg based on a body weight of 60 kg. The administration dose for mice was 10 times the clinical dose, approximately 0.02 g/kg. The used administration volume was 10 ml/kg and administration concentration was 0.02 g/10 ml. The drug was prepared using powder obtained by grinding the tablets, with each tablet containing 50 mg of Riluzole. During the preparation, one tablet was taken, ground with sterile injectable water and adjusted to a final volume of 25 ml. The obtained liquor can be used based on the number of animals.

The proposed clinical dose of TCM-073 was 18 g of raw drug/day. This test set three doses: low dose (1.5 g raw drug/kg), medium dose (3.0 g raw drug/kg), and high dose (6.0 g raw drug/kg), corresponding to 5, 10, and 20 times the clinical dose, respectively (See Table 14 for details). An appropriate amount of the drug was weighed and ground using sterile injectable water as the solvent to prepare high, medium, and low doses.

2.5 Administration of test product

The corresponding drug solution was administered via gavage using a syringe at a volume of 10 ml/kg. The model group and negative control group were administered with an equivalent volume of sterile injectable water. The administration dose for each animal was adjusted weekly according to the animal's latest weighed weight.

2.6 Determination of model mice

Based on preliminary experimental experience, literature review, and the disease characteristics of this mouse strain, the administration was started when the mice were 70 days old.

2.7 Indicators, time and contents of observation

After 30 days of administration, electrophysiological measurements were performed, and tissue material was collected for subsequent detections.

2.7.1 Electrophysiological parameters

Mice were anesthetized and placed in a prone position. A recording electrode was inserted into the gastrocnemius muscle belly of the hindlimb. The lumbar paraspinal sciatic nerve was stimulated using a stimulator. Peak value, wave amplitude and time course within region of interest of compound muscle action potentials were observed and recorded using a PowerLab physiological recorder.

2.7.2 Serum malondialdehyde (MDA)

Blood of mice was collected by removal of eyeball. The blood samples were allowed to stand until supernatant separated out, and then centrifuged at 3,000 rpm for 10 minutes at 4°C to isolate serum. The serum was aliquoted into EP tubes and stored in an ultra-low temperature refrigerator (-80°C). MDA content was measured. The inhibition rate on oxidative damage by each drug group was calculated according to MDA content, following the formula of:

$$\% \text{ Inhibition rate} = \frac{\text{MDA content of model control group} - \text{MDA content of administration group}}{\text{MDA content of model control group}} \times 100\%$$

$$\frac{\text{模型组 MDA 含量} - \text{给药组 MDA 含量}}{\text{模型组 MDA 含量}} \times 100\%$$

2.7.3 Histomorphological observation indicator

HE staining was carried out to observe gastrocnemius muscle and sciatic nerve. Toluidine blue staining was carried out to observe the morphology and number of motor neurons in the spinal cord.

2.7.4 Immunofluorescence

Based on the amount of spinal cord and muscle tissues harvested from mice, neuromuscular junction, stem cell regeneration, microglial senescence and degree of myelin and neuronal damage and repair were detected. For example, the muscular presynaptic marker (synaptotagmin-2) and the acetylcholine receptor (AChR) marker ($\alpha$-BTX) were co-stained, the satellite cell marker (Pax7) and laminin 2$\alpha$ were co-stained, the myelin marker (myelin basic protein, MBP) was stained, and the neuronal marker (beta-III Tubulin, also known as Tuj-1) was stained.

2.7.5 Molecular biological indicators

Based on the amount of spinal cord tissue harvested from mice, myelin- and synapse-related molecular biological indicators were detected. The myelin- and synapse-related indicators included myelin basic protein (MBP) and synaptic vesicle protein 2A (SV2A).

2.8 Notification of relevant staff

The animal department should be notified when animals were purchased. The abnormal event of animals should be reported to the pathology department for processing.

2.9 Statistical methods

The experimental data were presented as mean $\pm$ standard deviation ($\bar{x}\pm s$). The statistical analysis was performed using statistical software SPSS 25.0. Behavioral scores and tissues detection indicators were firstly subjected to a normality test. For data that followed a normal distribution, means were compared by one-way ANOVA. If the variances were homogeneous, pairwise comparisons were performed using the least significant difference method. If the variances were not homogeneous, Dunnett's T3 test was used. For data that did not follow a normal distribution, non-parametric tests were used for statistical analysis.

2.10 Instrument system

JJ2000 electronic balance from Changshu Shuangjie Testing Instrument Factory

Sartorius BT224S precision analytical balance from Sartorius Scientific Instruments (Beijing) Co. Ltd.

NVT2201ZH electronic balance from OHAUS Instrument (Shanghai) Co., Ltd.

Mettler ME104E analytical balance from Mettler-Toledo Instruments (Shanghai) Co., Ltd.

DT2000 electronic balance from Changshu Shuangjie Testing Instrument Factory

PowerLab physiological recorder from AD Instruments International Trade (Shanghai) Co., Ltd.

Inverted fluorescence confocal microscope (Zeiss)

Mettler electronic balance from Mettler-Toledo Instruments (Shanghai) Co., Ltd.

SYG-4 digital constant temperature water bath pot from Changzhou Langyue Instrument Manufacturing Co., Ltd.

Gene Speed 1580R high-speed refrigerated centrifuge from Gene Co., Ltd.

TECAN Infinite M200 PRO Microplate Reader (TECAN, Switzerland)

WS-350P microplate oscillator (Wiggens Labortechnik GmbH)

SE260 vertical electrophoresis unit (Ge Healthcare UK Ltd)

EPS301 electrophoresis powder supply (Ge Healthcare Bio-Sciences AB)

eBlot L1 fast wet transfer system from Genscript Biotechnology Co., Ltd.

WD-9405DN digital horizontal shaker from Beijing Liuyi Biotechnology Co., Ltd.

Odyssey clx 2-channel near-infrared laser imager (LI-COR)

Multi-sample tissue grinder from Shanghai Jingxin Industrial Development Co., Ltd.

ZWYR-240 constant temperature shaking incubator from Shanghai Zhicheng Analytical Instrument Manufacturing Co., Ltd.

CHB-202 constant temperature metal bath from Hangzhou Bioer Technology Co., Ltd.

BPN-CH $CO_2$ Incubator (Shanghai Yiheng Scientific Instrument Co., Ltd.)

3. Results

3.1 Effect on muscle action potential

As shown in Tables 15, 16 and 17, compared to the negative control group, the model control group exhibited a significantly reduced peak value (P<0.001), significantly reduced wave amplitude (P<0.001), and significantly prolonged time course within region of interest (P <0.001) of the action potential. Compared to the model control group, the positive drug group and each administration group exhibited a significantly increased peak value of action potential (P<0.05, P<0.01). Compared to the model control group, the positive drug group (P<0.05), TCM-073 low-dose group (P<0.05) and TCM-073 high-dose group (P<0.01) exhibited significantly increased wave amplitude of action potential. Compared to the model control group, the positive drug group (P<0.05) and each administration group (P<0.01) exhibited a significantly shortened time course within region of interest of action potential.

Table 15 Peak value of action potential

| Group | | No division by gender | | Male | | Female | |
|---|---|---|---|---|---|---|---|
| | | N | Peak value | N | Peak value | N | Peak value |
| Negative control group | | 20 | 13.8±2.2 | 10 | 14.6±2.5 | 10 | 13.0±1.7 |
| Model control group | | 16 | 6.5±2.5### | 8 | 7.3±2.9## | 8 | 5.8±2.0### |
| Positive drug control group (Riluzole) | | 16 | 9.6±3.3** | 8 | 8.8±2.2 | 8 | 10.4±4.0** |
| TCM-073 | Low dose | 16 | 8.9±2.1** | 8 | 8.0±1.7 | 8 | 9.8±2.3** |
| | Medium dose | 16 | 8.8±3.3** | 8 | 7.7±2.9 | 8 | 9.9±3.6** |
| | High dose | 16 | 9.3±3.0** | 8 | 7.6±2.3 | 8 | 10.9±2.7** |

### indicates P<0.001, * indicates P<0.05, and ** indicates P<0.01. # means a comparison between the negative control group and the model control group. * means a comparison between an administration group and the model control group.

Table 16 Wave amplitude of action potential

| Group | No division by gender | | Male | | Female | |
|---|---|---|---|---|---|---|
| | N | Wave amplitude | N | Wave amplitude | N | Wave amplitude |
| Negative control group | 20 | 20.4±3.4 | 10 | 21.8±3.6 | 10 | 19.0±2.8 |
| Model control group | 16 | 8.6±3.3### | 8 | 8.8±3.6### | 8 | 8.3±3.1### |

(continued)

| Group | | No division by gender | | Male | | Female | |
|---|---|---|---|---|---|---|---|
| | N | Wave amplitude | N | Wave amplitude | N | Wave amplitude | |
| Positive drug control group (Riluzole) | 16 | 13.0±5.0* | 8 | 11.2±3.9 | 8 | 14.8±5.6* | |
| TCM-073 Low dose | 16 | 11.4±2.8* | 8 | 10.4±2.0 | 8 | 12.3±3.2* | |
| TCM-073 Medium dose | 16 | 12.2±6.5 | 8 | 9.7±4.2 | 8 | 14.6±7.6* | |
| TCM-073 High dose | 16 | 12.4±4.4** | 8 | 9.3±3.6 | 8 | 15.5±2.6** | |

### indicates P<0.001, * indicates P<0.05, and ** indicates P<0.01. # means a comparison between the negative control group and the model control group. * means a comparison between an administration group and the model control group.

Table 17 Time course within region of interest of action potential

| Group | | No division by genderx | | Male | | Female | |
|---|---|---|---|---|---|---|---|
| | N | Time course within region of interest | N | Time course within region of interest | N | Time course within region of interest | |
| Negative control group | 20 | 0.0014±0.0001 | 10 | 0.0013±0.0001 | 10 | 0.0014±0.0001 | |
| Model control group | 16 | 0.0019±0.0003### | 8 | 0.0019±0.0003### | 8 | 0.0019±0.0002### | |
| Positive drug control group (Riluzole) | 16 | 0.0017±0.0002* | 8 | 0.0016±0.0001 | 8 | 0.0018±0.0002 | |
| TCM-073 Low dose | 16 | 0.0016±0.0002** | 8 | 0.0017±0.0003 | 8 | 0.0016±0.0001** | |
| TCM-073 Medium dose | 16 | 0.0016±0.0002** | 8 | 0.0016±0.0001* | 8 | 0.0017±0.0002 | |
| TCM-073 High dose | 16 | 0.0016±0.0002** | 8 | 0.0017±0.0001 | 8 | 0.0016±0.0003* | |

### indicates P<0.001, * indicates P<0.05, and ** indicates P<0.01. # means a comparison between the negative control group and the model control group. * means a comparison between an administration group and the model control group.

3.2 Effect on serum malondialdehyde (MDA) content

Blood of mice was collected by removal of eyeball. Blood samples were allowed to stand until supernatant separated out, and then centrifuged at 3,000 rpm for 10 minutes at 4°C to isolate serum. The MDA content was measured using a kit, and the inhibition rate on oxidative damage by each drug group was calculated according to MDA content. The results are shown in Table 18. Compared to the negative control group, the MDA content in the model control group was significantly increased (P<0.01). The positive drug group and each administration group significantly reduced MDA content (P<0.05, P<0.01). Wherein, compared to the positive drug group, the TCM-073 medium-dose group (P<0.05) and TCM-073 high-dose group (P<0.01) showed significantly reduced serum malondialdehyde content.

Table 18 Serum malondialdehyde (MDA) content

| Group | N | MDA (μM) | % Inhibition rate |
|---|---|---|---|
| Negative control group | 6 | 20.0±0.6 | 48.21 |
| Model control group | 6 | 38.7±1.3## | 0.00 |
| Positive drug control group (Riluzole) | 6 | 31.0±4.8* | 20.04 |

(continued)

| Group | | N | MDA (µM) | % Inhibition rate |
|---|---|---|---|---|
| TCM-073 | Low dose | 6 | 30.1±3.7** | 22.26 |
| | Medium dose | 6 | 22.7±4.5**& | 41.35 |
| | High dose | 6 | 21.8±0.6**&& | 43.79 |

## indicates $P<0.01$, * indicates $P<0.05$, ** indicates $P<0.01$, & indicates $P<0.05$ and && indicates $P<0.01$. # means a comparison between the negative control group and the model control group. * means a comparison between an administration group and the model control group. & means a comparison between the positive drug control group and an administration group.

3.3 Effects on hitomorphological observation indicators

HE staining of the sciatic nerve showed that the main pathological changes of animals in the model control group were axonal degeneration of the sciatic nerve accompanied by myelin degeneration, with slightly more severe lesions in male animals. Compared to the model control group, the pathological changes were slightly milder in the positive drug control group and the TCM-073 high-dose group. No significant improvement was observed in the other administration groups. See Table 19 for details.

Table 19 Pathological report

| | |
|---|---|
| Project Title: | Study on action mechanism of TCM-073 in treating SOD1-G93A transgenic mice with amyotrophic lateral sclerosis |
| Project Number: | TSN-2136 |
| Examination Sample: | Sciatic nerve samples obtained from 36 mice were fixed in 10% neutral formalin, embedded with paraffin, sectioned at a thickness of 4 µm, and subjected to HE staining. Experimental animals were divided into 6 groups: negative control group, model control group, positive drug control group, TCM-073 low-dose group, TCM-073 medium-dose group and TCM-073 high-dose group, 6 mice/group, half male and half female. |
| Examination Objective: | To observe histomorphology of sciatic nerves through HE staining. |
| Submitted by: | Wang Lu |
| Submission Date: | 2021.12.08 |
| Examination Result Grading | Lesion Grading: The grading based on the lesion area and severity degree was classified into mild (+), moderate (++), moderately severe (+++) and severe (++++). |
| Negative Control Group | Results of Microscopic Examination:<br>No obvious pathological changes were observed in the sciatic nerves of all 6 mice. See Table 20 for details. |
| Model Control Group | Results of Microscopic Examination:<br>All 6 sciatic nerves in 6 samples showed axonal degeneration with myelin degeneration (3 samples: +++, 3 samples: ++). Males exhibited slightly more severe lesions compared to females. See Table 20 for details. |
| Positive Drug Control Group | Results of Microscopic Examination:<br>All 6 sciatic nerves in 6 samples showed axonal degeneration with myelin degeneration (1 sample: +++, 4 samples: ++, 1 sample: +).<br>No difference in degree of lesion between males and females. See Table 20 for details. |
| TCM-073 Low-Dose Group | Results of Microscopic Examination:<br>All 6 sciatic nerves in 6 samples showed axonal degeneration with myelin degeneration (4 samples: +++, 2 samples: ++).<br>No difference in degree of lesion between males and females. See Table 20 for details. |

(continued)

| | Results of Microscopic Examination: |
|---|---|
| TCM-073 Medium-Dose Group | All 6 sciatic nerves in 6 samples showed axonal degeneration with myelin degeneration (3 samples: +++, 3 samples: ++). Males exhibited slightly more severe lesions compared to females. See Table 20 for details. |
| TCM-073 High-Dose Group | All 6 sciatic nerves in 6 samples showed axonal degeneration with myelin degeneration (2 samples: +++, 4 samples: ++). No difference in degree of lesion between males and females. See Table 20 for details. |

Conclusion

In summary, the pathological examinations of this test showed that animals in the model control group developed predominant lesions of axonal degeneration with myelin degeneration in the sciatic nerve, with slightly more severe lesions observed in male animals. Compared to the model control group, the positive drug control group and the TCM-073 high-dose group showed slightly milder lesions. No obvious improvement was observed in the other administration groups. See Table 20 for details.

Table 20 Results of major lesions scoring of individual animal

| Group | Sex | Animal Number | Axonal degeneration with myelin degeneration | Score (divided by gender) | Group Score |
|---|---|---|---|---|---|
| Negative control group | Male | 203 | - | 0 | 0 |
| | | 204 | - | | |
| | | 206 | - | | |
| | Female | 251 | - | 0 | |
| | | 252 | - | | |
| | | 253 | - | | |
| Model control group | Male | 215 | +++ | 3 | 2.5 |
| | | 219 | ++ | | |
| | | 224 | +++ | | |
| | Female | 277 | ++ | 2 | |
| | | 278 | +++ | | |
| | | 283 | ++ | | |
| Positive drug control group | Male | 217 | ++ | 2 | 2 |
| | | 218 | +++ | | |
| | | 232 | ++ | | |
| | Female | 280 | + | 2 | |
| | | 287 | ++ | | |
| | | 294 | ++ | | |
| TCM-073 low-dose group | Male | 222 | ++ | 3 | 3 |
| | | 226 | +++ | | |
| | | 239 | +++ | | |
| | Female | 266 | +++ | 3 | |
| | | 276 | +++ | | |
| | | 288 | ++ | | |

(continued)

| Group | Sex | Animal Number | Axonal degeneration with myelin degeneration | Score (divided by gender) | Group Score |
|---|---|---|---|---|---|
| TCM-073 medium-dose group | Male | 221 | +++ | 3 | 2.5 |
| | | 229 | ++ | | |
| | | 244 | +++ | | |
| | Female | 274 | ++ | 2 | |
| | | 285 | +++ | | |
| | | 295 | ++ | | |
| TCM-073 high-dose group | Male | 216 | ++ | 2 | 2 |
| | | 230 | ++ | | |
| | | 245 | +++ | | |
| | Female | 284 | ++ | 2 | |
| | | 290 | +++ | | |
| | | 297 | ++ | | |

Note 1: Lesions were graded into no lesion (-), mild (+), moderate (++), moderately severe (+++) and severe (++++).
Note 2: Group scores were calculated based on the median of the score of lesion grade of animals in each group ("-" represents 0 points, each "+" represents 1 point, and higher scores indicate more severe lesions).

3.4 Effect on neuromuscular junction, muscle, damage and repair of myelin and neuron

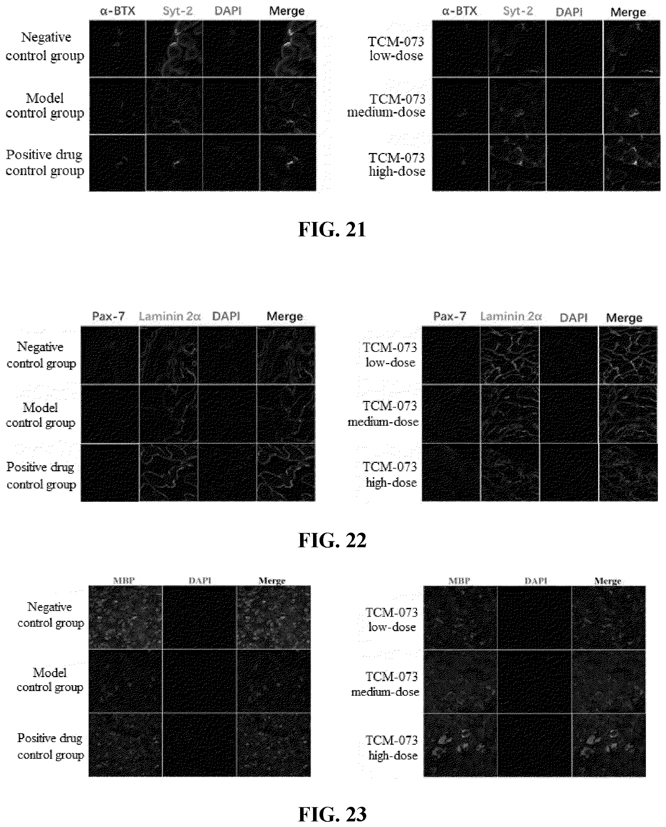

The muscular presynaptic marker (synaptotagmin-2) and acetylcholine receptor (AChR) marker ($\alpha$-BTX) of the gastrocnemius muscle were detected by the immunofluorescence. The results are shown in FIG. 21. The model control group showed reduced co-localization degree of synaptotagmin-2 and $\alpha$-BTX compared to the negative control group. The positive drug control group and each administration group showed increased co-localization degree.

The satellite cell marker (Pax7) and laminin $2\alpha$ of the gastrocnemius muscle were detected by the immunofluorescence. The results are shown in FIG. 22. The model control group showed a decreased number of Pax7-positive cells compared to the negative control group. The positive drug control group and each administration group showed an increased number of Pax7-positive cells and Laminin $2\alpha$ delineated the cellular boundaries.

The myelin marker (myelin basic protein, MBP) of the spinal cord was detected by the immunofluorescence. The results are shown in FIG. 23. The model control group showed reduced MBP expression compared to the negative control group. The positive drug control group and each administration group showed increased MBP expression.

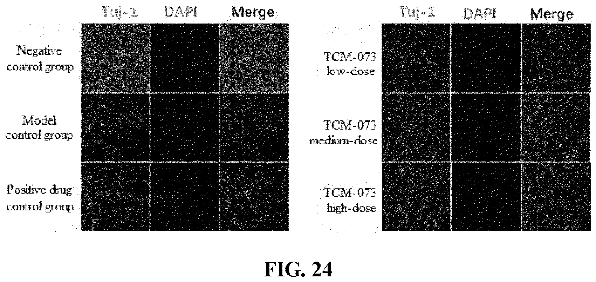

The neuronal marker (beta-III Tubulin, also known as Tuj-1) of the spinal cord was detected by the immunofluorescence. The results are shown in FIG. 24. The model control group showed reduced Tuj-1 expression compared to the negative control group. The positive drug control group and each administration group showed increased Tuj-1 expression.

3.5 Effect on spinal myelin- and synapse-related indicators in molecular biological detection

Based on the amount of spinal cord tissue harvested from mice, myelin- and synapse-related molecular biological indicators, including myelin basic protein (MBP) and synaptic vesicle protein 2A (SV2A), were detected. The results are shown in FIGs. 21 and 22. The results of immunoblotting assay showed that the model control group exhibited significantly reduced MBP expression compared to the negative control group ($P<0.05$). The positive drug and each administration group showed significantly increased MBP content ($P<0.05$). The model control group showed significantly reduced SV2A expression compared to the negative control group ($P<0.001$). The TCM-073 high-dose group was found to increase SV2A content ($P<0.01$), which was significantly higher than that of the positive drug control group ($P<0.01$).

Table 21 MBP content in spinal cord

| Group | N | MBP |
|---|---|---|
| Negative control group | 4 | $1.00\pm0.00$ |
| Model control group | 4 | $0.36\pm0.03\#$ |
| Positive drug control group (Riluzole) | 4 | $0.99\pm0.62*$ |
| TCM-073 Low dose | 4 | $0.80\pm0.36*$ |
| TCM-073 Medium dose | 4 | $0.64\pm0.15*$ |
| TCM-073 High dose | 4 | $0.96\pm0.24*$ |

# indicates P<0.05 and * indicates P<0.05. # means a comparison between the negative control group and the model control group. * means a comparison between an administration group and the model control group.

Table 22 SV2A content in spinal cord

| Group | N | SV2A |
|---|---|---|
| Negative control group | 4 | $1.00\pm0.00$ |
| Model control group | 4 | $0.31\pm0.16\#\#\#$ |
| Positive drug control group (Riluzole) | N 4 | $0.41\pm0.15$ |
| TCM-073 Low dose | 4 | $0.39\pm0.15$ |
| TCM-073 Medium dose | 4 | $0.53\pm0.27$ |
| TCM-073 High dose | 4 | $0.79\pm0.16**\&\&$ |

### indicates P<0.001, ** indicates P<0.01, and && indicates P<0.01. # means a comparison between the negative control group and the model control group. * means a comparison between an administration group and the model control group. & means a comparison between the positive drug control group and an administration group.

4 Conclusion

In this test, SOD1-G93A transgenic mice as a classic ALS model were used, administered at 70 days old and observed to evaluate the therapeutic effects of various doses of TCM-073. The results showed that all dose groups of the test product increased the peak value of muscle action potential and shortened the time course within region of interest. The low-dose (1.5 g raw drug/kg) and high-dose (6.0 g raw drug/kg) groups of test product increased the wave amplitude of action potential. Each administration group significantly reduced serum malondialdehyde content (P<0.01), and the medium-dose (3.0 g raw drug/kg) and high-dose (6.0 g raw drug/kg) TCM-073 groups showed significantly greater effects than the positive drug (P<0.05, P<0.01). HE staining of the sciatic nerve showed that the positive drug control group and the TCM-073 high-dose group (6.0 g raw drug/kg) had slightly milder lesions compared to the model control group. No significant improvement was observed in the other administration groups. The results of immunofluorescence assay showed that the positive drug control group and each administration group increased the co-localization degree of neuromuscular junctions, increased the number of positive satellite cells in the gastrocnemius muscle, and increased the expression levels of the myelin marker and neuronal marker. The results of immunoblotting assay showed that the positive drug control group and each administration group significantly increased MBP content in spinal cord (P<0.05). The TCM-073 high-dose group (6.0 g raw drug/kg) significantly increased SV2A content (P<0.01), which was significantly higher than the positive drug control group (P<0.01).

5 Record and storage

Except for data collected directly by computers or automated instruments, all other data obtained in actual research were recorded on forms or record sheets and organized and bound in time. All recorded data were signed by the operator and verifier, and the date of recording was noted. When the final report was submitted, all original information, documents, and other relevant materials were submitted to the archive room for storage.

5.1 Storage unit: Innovative Drug Evaluation Center of Hebei Yiling Pharmaceutical Research Institute

5.2 Address: No. 238, Tianshan Street, Shijiazhuang

Experimental example 5: Study on action mechanism of the pharmaceutical composition of the present invention in treating demyelination model mice induced by CPZ

Experimental objective

The CPZ-induced demyelination model mice were used to assess the therapeutic effect of the pharmaceutical composition of the present invention on demyelination of the central nervous system.

1. Experimental materials

1.1 Test product: The pharmaceutical composition of the present invention was prepared as in Example 2, which was abbreviated as TMC-073.

1.2 Main reagents:

Sterile water for injection from Shijiazhuang No. 4 Pharmaceutical Co. Batch number 2009053201. Expiration date: 2022.09.04.

1.3 Experimental system

1.3.1 Animal Strain: C57BL/6J
1.3.2 Animal Grade: SPF grade.
1.3.3 Animal Gender and Quantity: 60 male animals, from which 58 animals were selected for testing.
1.3.4 Age of animals: 42-48 days old.
1.3.5 Weight of animals at the start of dosing: 24.1-31.7 g.
1.3.6 Animal Source: Beijing Vital River Laboratory Animal Technology Co., Ltd.
1.3.7 Animal Certification of Quality, issuing authority, and receipt date: Certification number: 110011220103270315. License number SCXK (Beijing) 2021-0006. Issuing authority: Beijing Vital River Laboratory Animal Technology Co., Ltd. Receipt date: March 30, 2022.
1.3.8 Feeding Conditions: Mice were reared in cages at the Innovative Drug Evaluation Center of Hebei Research Institute of Integrated Traditional Chinese and Western Medicine. License Number: SYXK (Hebei) 2020-006. Lighting: 12 h/day. Temperature: 20 to 26°C. Relative Humidity: 40% to 70%.
1.3.9 Quarantine process: Animals were subjected to adaptive feeding for at least 5 days. During this period, whether the animals had normal water and food intake, and whether the animals were in good health status, had illness and showed signs of mortality were observed.
1.3.10 Feed: Rat and mice maintenance feed (SPF-grade) was provided by SPF (Beijing) biotechnology Co. Ltd. Experimental Animal Production License Number: SCXK (Beijing) 2019-0010. Product Batch Number: 0112SH01140404C. Expiration date: 2022.07.13. 0.2% cuprizone-containing feed was provided by Xiaoshu Youtai (Beijing) Biotechnology Co., Ltd. Experimental Animal Production License Number: SCXK (Beijing) 2018-0006. Production Date: 2022.03.14. Expiration date: 2022.06.13.
1.3.11 Water: Clean water prepared by the ROB-B drinking water system for laboratory animal was provided, and filled in water bottles for free drinking. The drinking water was detected annually for all indicators, including mainly microbiological indicators, toxicological indicators, sensory characteristics, and general chemical parameters, and the detection was commissioned to a qualified institution for detection. Microbial limit detection was performed every 2 months, which was commissioned to the Quality Control Department of Shijiazhuang Yiling Pharmaceutical Co., Ltd. The detection reports were stored by the animal department and archived annually.
1.3.12 Bedding: Corn cob bedding (common grade) provided by Beijing Keao Xieli Feed Co., Ltd., was sterilized by high temperature and high pressure before use. Experimental Animal Production License Number: SCXK (Beijing) 2020-0010. Batch Numbers: 22019811 and 22049811.
1.3.13 Marking: Mice were marked with ear tags, provided by Globalebio (Beijing) Technology Co., Ltd.

2 Test methods

2.1 Test design basis

2.1.1 Adopted standards were determined according to the Registration, Classification and Submission Data Requirements for Chinese Traditional Medicine issued by the State Food and Drug Administration, Good Laboratory Practice for non-clinical laboratory studies, and related literatures.

2.1.2 Description of selected experimental system: Oral intake of cuprizone (CPZ) can induce oligodendrocyte apoptosis within a few days, followed by the activation of innate immune cells in brain (i.e., astrocytes and microglia), ultimately leading to demyelination in distinct white and gray matter brain areas. Therefore, this model thus reflects several important characteristics of the progressive demyelination disease course. The CPZ-induced demyelination model can be used for developing drugs and investigating mechanisms related to demyelination pathologies. Estrogen has been shown in the literature to have a protective effect against demyelination induced by CPZ. Therefore, only male animals were used for modeling in this test. Mice aged 6-8 weeks were orally given feeds containing 0.2% CPZ for 6 weeks to induce acute demyelination. This was followed by normal feeds for 2 weeks to allow spontaneous and endogenous myelin regeneration in mice, during which the test drug was given to evaluate whether it can accelerate this myelin regeneration process.

2.2 Dosage and grouping

Mice were given 0.2% CPZ containing-feeds for 6 weeks. After the results of Weaver's 15-point scale showed a significant difference between them and the mice in solvent control group, the random grouping was carried out according to the scores and body weight. The administration and observation were started. Specifically, 10 mice were included in solvent control group (C), and 12 mice were included in each of model control group (M), TCM-073 low-dose group (073-L), TCM-073 medium-dose group (073-M) and TCM-073 high-dose group (073-H). Specific experimental groupings and doses are shown in Table 23.

Table 23 Experimental grouping and dose setting

| Group | | Number of animals | Dose (g raw drug/kg) | Specification(g raw drug/g powder) | Dose for formulation (g powder/kg) | Formulation concentration (g powder/ml) | Multiple relative to clinical equivalent dose |
|---|---|---|---|---|---|---|---|
| Solvent control group | | 10 | - | - | - | - | - |
| Model control group | | 10 | - | - | - | - | - |
| TCM-073 | Low dose | 10 | 1.50 | 3.1 | 0.48 | 0.048 | 5 |
| | Medium dose | 10 | 3.00 | 3.1 | 0.97 | 0.097 | 10 |
| | High dose | 10 | 6.00 | 3.1 | 1.94 | 0.194 | 20 |

2.3 Administration method

The drug was orally administered via gavage, consistent with the clinically recommended oral administration.

2.4 Preparation and storage of test product

The proposed clinical dose of TCM-073 was 18 g of raw drug/day. This test set three doses: low dose (1.5 g raw drug/kg), medium dose (3.0 g raw drug/kg), and high dose (6.0 g raw drug/kg), corresponding to 5, 10, and 20 times the clinical dose, respectively (see Table 23 for details). An appropriate amount of the drug was weighed and ground using sterile injectable water as the solvent to prepare high, medium, and low doses.

2.5 Administration of test product

The corresponding drug solution was administered via gavage using a syringe at a volume of 10 ml/kg. The model group and solvent control group were administered with an equivalent volume of sterile injectable water.

2.6 Determination of model mice

2.6.1 Weaver's 15-Point Scoring System: Tail movement-performance was classified into 3 grades, and limb movement-performance was classified into 4 grades. The cumulative score was obtained through summing points (See Table 24). A death event yields 15 points. Literature review indicates that after 6 weeks of intake of 0.2% CPZ-containing feeds, mice exhibited symptoms of acute demyelination.

Table 24 Weaver's 15-Point Scoring

| Observation site | Clinical symptom | Score |
| --- | --- | --- |
| Tail | No symptoms | 0 |
| | Reduced tail tone or partial paralysis of the tail | 1 |
| | Full paralysis of the tail | 2 |
| Four limbs | No symptoms | 0 |
| | Unstable gait | 1 |
| | Mild limb paralysis with dragging limbs during walking | 2 |
| | Full limb paralysis with outwardly flipped limbs during walking | 3 |

Note: The limbs and tail were scored individually based on the severity of paralysis, and the cumulative score was obtained by summation

2.7 Indicators, time and contents of observation

During the administration period in the test, neurological function was evaluated weekly using the Weaver's 15-point scoring system. The rotarod test and open field test were conducted after 14 days of administration.

2.7.1 Rotarod test

The rotarod test was performed to assess sensorimotor coordination in each mouse. The rotarod was set to rotate at a speed of 30 rpm for 120 seconds. The duration that each mouse remained on the rotarod was recorded.

2.7.2 Open field test

The open field apparatus consisted of an open field box and an automated data acquisition and process system. A camera was set directly above the open field box to monitor the entire area within the open field. The mouse was firstly placed in the central zone of the open field box, and video recording and timing were started simultaneously. The total distance spontaneously traveled by the mouse during a 5-minute period was recorded.

2.8 Notification of relevant staff

The animal department should be notified when animals were purchased. The abnormal event of animals should be reported to the pathology department for processing.

2.9 Statistical methods

The experimental data were presented as mean $\pm$ standard deviation ($\bar{x}\pm s$). The statistical analysis was performed using statistical software SPSS 25.0. Detection indicators of tissues and behavioral scores were firstly subjected to a normality test. For data that followed a normal distribution, means were compared by one-way ANOVA. If the variances were homogeneous, pairwise comparisons were performed using the Least Significant Difference method. If the variances were not homogeneous, Dunnett's T3 test was used. For data that did not follow a normal distribution, non-parametric tests were used for statistical analysis.

2.10 Instrument system

Sartorius BSA2201 electronic balance from Sartorius Scientific Instruments (Beijing) Co. Ltd.

Sartorius BT224S precision analytical balance from Sartorius Scientific Instruments (Beijing) Co. Ltd.

IITC Rotarod unit for mice and rats from IITC Life Science

ANY-maze behavior tracking instrument for small animals from Jiangsu SANS Biotechnology Co., Ltd.

3 Results

3.1 Effect on neurological functions

As shown in Table 25, the model control group showed a significantly increased neurological function score on day 7 and day 14 of the administration compared to the solvent control group (P<0.001). No reduction in neurological function score was observed in each administration group on day 7. On day 14, the TCM-073 medium-dose (P<0.05) and TCM-073 high-dose group (P<0.01) showed a significantly reduced compared to the model control group.

Table 25 Neurological function score

| Group | | N | Neurological function score on day 7 | Neurological function score on day 14 |
|---|---|---|---|---|
| Solvent control group | | 10 | 0±0 | 0±0 |
| Model control group | | 12 | 1.25±0.45### | 1.50±0.52### |
| TCM-073 | Low dose | 12 | 1.25±0.45 | 1.08±0.51 |
| | Medium dose | 12 | 1.17±0.39 | 0.83±0.58* |
| | High dose | 12 | 1.08±0.51 | 0.58±0.51** |

### indicates P<0.001, * indicates P<0.05, and ** indicates P<0.01. # means a comparison between the solvent control group and the model control group. * means a comparison between an administration group and the model control group.

3.2 Effect on the duration that the mouse remained on the rotarod

The rotarod test was performed to assess sensorimotor coordination of each mouse. The rotarod was set to rotate at a speed of 30 rpm for 120 seconds. The duration that each mouse remained on the rotarod was recorded. The results are shown in Table 26. The model control group showed a significantly shortened duration that the mouse remained on the rotarod compared to the solvent control group (P<0.001), and each administration group significantly prolonged the duration that the mouse remained on the rotarod (P<0.05, P<0.01).

Table 26 Duration that mouse remained on the rotarod

| Group | | N | Duration that mouse remained on the rotarod (s) |
|---|---|---|---|
| Solvent control group | | 10 | 118.30±3.53 |
| Model control group | | 12 | 55.00±28.98### |
| TCM-073 | Low dose | 12 | 87.42±29.94* |
| | Medium dose | 12 | 90.67±28.56* |
| | High dose | 12 | 104.08±26.71** |

### indicates P<0.001, * indicates P<0.05, and ** indicates P<0.01. # means a comparison between the solvent control group and the model control group. * means a comparison between an administration group and the model control group.

3.3 Effect on open field test

The mouse was placed in the central zone of the open field box, and video recording and timing were started simultaneously. The total distance spontaneously traveled by the mouse, average speed, and a number of rotations during a 5-minute period were recorded. In mice demyelination model, the demyelination led to abnormal neural signal conduction, which was manifested as increased spontaneous activity, increased number of rotations, and other signs of anxiety or irritability. The results are shown in Table 27. Compared to the solvent control group, the model control group showed a significantly longer travel distance (P<0.05), a significantly higher average speed (P<0.05), and a significantly greater number of rotations (P<0.05). Each administration group showed a significant reduction in travel distance, decreased average speed, and lower number of rotaions (P<0.05, P<0.01).

Table 27 Results of open field test

| Group | N | Distance | Average speed | Number of rotations |
|---|---|---|---|---|
| Solvent control group | 8 | 22.60±6.32 | 0.038±0.011 | 22.50±8.78 |
| Model control group | 10 | 32.10±8.10# | 0.054±0.013# | 33.90±6.62# |
| TCM-073 Low dose | 12 | 23.76±7.27* | 0.040±0.012* | 25.00±7.73* |
| Medium dose | 12 | 23.13±8.15* | 0.039±0.014* | 26.08±6.44* |
| High dose | 12 | 22.08±4.83** | 0.037±0.008** | 22.58±2.57** |

### indicates $P<0.001$, * indicates $P<0.05$, and ** indicates $P<0.01$. # means a comparison between the solvent control group and the model control group. * means a comparison between an administration group and the model control group.

4 Conclusion

The test used CPZ-induced demyelination mice model to evaluate the therapeutic effects of various doses of the pharmaceutical composition of the present invention. The results showed that the medium- and high-dose groups of the test drug significantly reduced the neurological function score ($P<0.05$, $P<0.01$). For behavioral indicators, each administration group of the test drug significantly prolonged the duration that the mouse remained on the rotarod, reduced the traveled distance in open field test, decreased average speed, and reduced the number of rotations ($P<0.05$, $P<0.01$). This suggests that the pharmaceutical composition of the present invention has a protective effect against nerve function damage caused by demyelination.

5. Record and storage

Except for data collected directly by computers or automated instruments, all other data obtained in actual research were recorded on forms or record sheets and organized and bound in time. All recorded data were signed by the operator and verifier, and the date of recording was noted. When the final report was submitted, all original information, documents, and other relevant materials were submitted to the archive room for storage.

5.1 Storage unit: Innovative Drug Evaluation Center of Hebei Yiling Pharmaceutical Research Institute

5.2 Address: No. 238, Tianshan Street, Shijiazhuang

5.3 Postal code: 050035

The above description is only preferred embodiments of the present invention and is not intended to limit the present invention. Any modifications, equivalent replacements or improvements that are made in accordance with the principles and spirit of the present invention should fall within the protection scope of the present invention.

**Claims**

1.  A traditional Chinese medicine composition for use in treating amyotrophic lateral sclerosis, consisting of the following components in parts by weight: 3 to 9 parts of *Ginseng Radix et Rhizoma,* and 3 to 9 parts of *Cistanche deserticola.*

2.  The traditional Chinese medicine composition according to claim 1, wherein the traditional Chinese medicine composition consists of the following components in parts by weight: 4 to 6 parts of *Ginseng Radix et Rhizoma,* and 4 to 6 parts of *Cistanche deserticola.*

3.  The traditional Chinese medicine composition according to claim 1, wherein the traditional Chinese medicine composition consists of the following components in parts by weight: 3 parts of *Ginseng Radix et Rhizoma,* and 9 parts of *Cistanche deserticola.*

4.  The traditional Chinese medicine composition according to claim 1, wherein the traditional Chinese medicine composition consists of the following components in parts by weight: 6 parts of *Ginseng Radix et Rhizoma,* and 6 parts of *Cistanche deserticola.*

**5.** The traditional Chinese medicine composition according to claim 1, wherein the traditional Chinese medicine composition consists of the following components in parts by weight: 9 parts of *Ginseng Radix et Rhizoma,* and 3 parts of *Cistanche deserticola.*

**6.** The traditional Chinese medicine composition according to claim 1, wherein the traditional Chinese medicine composition consists of the following components in parts by weight: 7 parts of *Ginseng Radix et Rhizoma,* and 4 parts of *Cistanche deserticola.*

**7.** A method for producing the traditional Chinese medicine composition according to any one of claims 1 to 6, comprising producing an active ingredient of the traditional Chinese medicine composition by steps of:
weighing *Ginseng Radix et Rhizoma* and *Cistanche deserticola* according to a prescribed amount, adding water, decocting, filtering to obtain a filtrate, and concentrating the filtrate under reduced pressure to obtain an extract as the active ingredient of the traditional Chinese medicine composition.

**8.** Use of the traditional Chinese medicine composition according to any one of claims 1 to 6 or the traditional Chinese medicine composition produced by the method according to claim 7 in the manufacture of a medicament for treating amyotrophic lateral sclerosis.

**9.** The use according to claim 8, wherein the amyotrophic lateral sclerosis is manifested as one or more of muscle weakness, muscle atrophy, muscle twitching, muscle stiffness or flaccidity, dysarthria, difficulty in swallowing, shortness of breath, soreness and weakness of waist and knees, fatigue and laziness to speak, or fear of coldness and cold limbs.

**10.** Use of the traditional Chinese medicine composition according to any one of claims 1 to 6 in the manufacture of a medicament for delaying onset of paralysis in amyotrophic lateral sclerosis, improving impaired neurological function, alleviating sciatic nerve lesion, enhancing co-localization degree of neuromuscular junction.

**11.** Use of the traditional Chinese medicine composition according to any one of claims 1 to 6 in the manufacture of a medicament for prolonging hanging time of mice, increasing peak value of muscle action potential, shortening time course within region of interest.

**12.** Use of the traditional Chinese medicine composition according to any one of claims 1 to 6 in the manufacture of a medicament for ameliorating demyelination of central nervous system, increasing muscle strength and muscle fiber fullness, increasing blood supply to muscle tissue.

**13.** Use of the traditional Chinese medicine composition according to any one of claims 1 to 6 in the manufacture of a medicament for treating sarcopenia.

**14.** Use of the traditional Chinese medicine composition according to any one of claims 1 to 6 in the manufacture of a medicament for improving neuroelectrophysiology, reducing serum malondialdehyde level, promoting axonal growth and regeneration of myelin, protecting neurological function, enhancing co-localization degree of a muscular presynaptic marker and an acetylcholine receptor marker, increasing a number of positive satellite cells of gastrocnemius muscle and myelin basic protein (MBP) level, increasing structural integrity of neuromuscular junction, increasing expression of a neuronal marker (Tuj-1) and synaptic vesicle protein 2A (SV2A), promoting regeneration of myelin, improving behavioral change caused by demyelination, improving signal transmission from motor neuron to muscle, slowing progression of amyotrophic lateral sclerosis.

**15.** A medicament comprising the traditional Chinese medicine composition according to any one of claims 1 to 6 or the traditional Chinese medicine composition produced by the method according to claim 7.

**16.** The medicament according to claim 15, wherein the medicament is in a dosage form of capsule, tablet, granule, injection, pill, powder, spray or oral liquid.

**17.** The medicament according to claim 16, wherein the granule is produced by steps of:

(1) weighing *Ginseng Radix et Rhizoma* and *Cistanche deserticola* according to a prescribed amount, adding water, decocting to obtain a water extract, filtering the water extract to obtain a filtrate, and concentrating the filtrate to obtain an extract for later use;

(2) spray drying the extract using a spray dryer, and collecting spray powders for later use; and

(3) adding an appropriate amount of excipient to the spray powders obtained in step (2), and performing granulation according to a conventional method to obtain the granule.

18. The medicament according to claim 16, wherein the granule is produced by steps of:

(1) weighing *Ginseng Radix et Rhizoma* and *Cistanche deserticola* according to a prescribed amount, adding water, decocting three times with 10 times the amount of water for a first decoction and 8 times the amount of water for each of a second and third decoctions, 1.5 h each time, filtering to obtain a filtrate, concentrating the filtrate under reduced pressure to a relative density of 1.05 to 1.10 at 60°C to obtain an extract for later use;
(2) spray drying the extract using a spray dryer, and collecting spray powders for later use; and
(3) adding an appropriate amount of excipient to the spray powders obtained in step (2), and performing granulation according to a conventional method to obtain the granule.

19. A method for treating a disease in a subject in need thereof, comprising administering the traditional Chinese medicine composition according to any one of claims 1 to 6, the traditional Chinese medicine composition produced by the method according to claim 7 or the medicament according to any one of claims 15 to 18 to the subject in need thereof.

20. The method according to claim 19, wherein the treating includes at least one of:

delaying the onset of paralysis in amyotrophic lateral sclerosis, improving impaired neurological function, alleviating sciatic nerve lesion, enhancing co-localization degree of neuromuscular junction;
prolonging hanging time of mice, increasing peak value of muscle action potential, shortening time course within region of interest;
ameliorating demyelination of central nervous system, increasing muscle strength and muscle fiber fullness, increasing blood supply to muscle tissue;
improving neuroelectrophysiology, reducing serum malondialdehyde level, promoting axonal growth and regeneration of myelin, protecting neurological function, enhancing co-localization degree of a neuromuscular presynaptic marker and an acetylcholine receptor marker, increasing a number of positive satellite cells of gastrocnemius and myelin basic protein (MBP) level, increasing structural integrity of neuromuscular junction, increasing expression of a neuronal marker (Tuj-1) and synaptic vesicle protein 2A (SV2A), promoting regeneration of myelin, improving behavioral change caused by demyelination, improving signal transmission from motor neuron to muscle, and slowing progression of amyotrophic lateral sclerosis.

FIG. 1

**FIG. 2**

**FIG. 3**

FIG. 4

**FIG. 5**

**FIG. 6**

**FIG. 7**

**FIG. 8**

**FIG. 9**

**FIG. 10**

**FIG. 11**

**FIG. 12**

**FIG. 13**

**FIG. 14**

**FIG. 15**

**FIG. 16**

**FIG. 17**

**FIG. 18**

**FIG. 19**

**FIG. 20**

FIG. 21

FIG. 22

FIG. 23

**FIG. 24**

## INTERNATIONAL SEARCH REPORT

| International application No. |
| --- |
| **PCT/CN2024/075125** |

| A. | CLASSIFICATION OF SUBJECT MATTER |
| --- | --- |

A61K36/64(2006.01)i; A61K36/258(2006.01)i; A61K9/14(2006.01)i; A61K9/20(2006.01)i; A61P21/04(2006.01)i

According to International Patent Classification (IPC) or to both national classification and IPC

| B. | FIELDS SEARCHED |
| --- | --- |

Minimum documentation searched (classification system followed by classification symbols)

IPC:A61K; A61P

Documentation searched other than minimum documentation to the extent that such documents are included in the fields searched

Electronic data base consulted during the international search (name of data base and, where practicable, search terms used)

CNABS, TWABS, CNTXT, TWTXT, DWPI, PUBMED, CNKI, 读秀, DUXIU, 超星, CHAOXING, E药全库, E MEDICINE LIBRARY, 肌萎灵, 人参, 肉苁蓉, 人参皂苷, 苯乙醇苷, 松果菊苷, 异类叶升麻苷, 毛蕊花糖苷, 水, 提取, 浓缩, 浸膏, 胶囊, 颗粒, 肌萎缩侧索硬化症, 渐冻症, 肌少症, Jiweiling, Ginseng, Cistanche+, Ginsenoside, phenylethanoside, echinacoside, isophylloside, verbascoside, water, extraction, concentration, extract, capsule, granule, Amyotrophic lateral sclerosis, ALS, Freezing disorder, Sarcopenia

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | CN 111281908 A (QINGWA WANGZI (FUJIAN) BABY CARE PRODUCTS CO., LTD. et al.) 16 June 2020 (2020-06-16) abstract, and description, paragraphs 60 and 88-97 | 1-5, 15 |
| Y | 陈金亮 等 (CHEN, Jinliang et al.). "肌萎灵注射液治疗运动神经元病的效果观察 (Effective Observation of the Parenteral Solution of Jiweiling on Motor Neuron Disease)" 中国临床康复 (Chinese Journal of Clinical Rehabilitation), Vol. 8, No. (35), 15 December 2004 (2004-12-15), page 7928, left-hand colum, table 1, and right-hand column, tables 2 and 3, and paragraph 2 | 1-18 |
| Y | CN 112236156 A (GREEN CROSS WELLBEING CORPORATION) 15 January 2021 (2021-01-15) description, paragraphs [0014]-[0017] | 1-18 |
| Y | CN 106955297 A (SINPHAR TIAN-LI PHARMACEUTICAL CO., LTD. (HANGZHOU)) 18 July 2017 (2017-07-18) description, paragraphs 10, 45 and 90 | 1-18 |

☑ Further documents are listed in the continuation of Box C.　　☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **29 March 2024** | **19 April 2024** |

| Name and mailing address of the ISA/CN | Authorized officer |
| --- | --- |
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| | International application No. |
|---|---|
| | **PCT/CN2024/075125** |

**C.    DOCUMENTS CONSIDERED TO BE RELEVANT**

| Category* | Citation of document, with indication, where appropriate, of the relevant passages | Relevant to claim No. |
|---|---|---|
| X | CN 112891400 A (INNER MONGOLIA AUTONOMOUS REGION ACADEMY OF AGRICULTURAL & ANIMAL HUSBANDRY SCIENCES) 04 June 2021 (2021-06-04) description, paragraph 59, and embodiment 3 | 1, 2, 4, 15 |
| Y | CN 1466958 A (ZHANG, Xiaoping) 14 January 2004 (2004-01-14) description, page 1, paragraphs 1, 3 and 5 | 1-18 |
| Y | CN 114796295 A (FAMENITY CO., LTD.) 29 July 2022 (2022-07-29) claims 1-6, and description, paragraphs 9 and 64 | 1-18 |
| Y | JIANG, F. et al. "Beneficial Effect of Ginseng Root in SOD-1 (G93A) Transgenic Mice." *Journal of the Neurological Sciences*, No. no. 180, 30 November 2000 (2000-11-30), p. 52, abstract | 1-18 |
| Y | 潘卫东 等 (PAN, Weidong et al.). "加味四君子汤对肌萎缩侧索硬化症AR2小鼠运动功能的影响 (Effects of "Jiawei Sijunzi Decoction" on Movement Dysfunction of AR2 Mice with Amyotrophic Lateral Sclerosis)" 上海中医药大学学报 *(Academic Journal of Shanghai University of Traditional Chinese Medicine)*, Vol. 27, No. (1), 25 January 2013 (2013-01-25), page 61, left-hand column, paragraph 2 | 1-18 |
| A | CN 1785223 A (HEBEI YILING PHARMACEUTICAL RESEARCH INSTITUTE CO., LTD.) 14 June 2006 (2006-06-14) abstract, and claims 1-7 | 1-18 |

Form PCT/ISA/210 (second sheet) (July 2022)

## INTERNATIONAL SEARCH REPORT

| International application No. |
| --- |
| **PCT/CN2024/075125** |

**Box No. II    Observations where certain claims were found unsearchable (Continuation of item 2 of first sheet)**

This international search report has not been established in respect of certain claims under Article 17(2)(a) for the following reasons:

1. ☑    Claims Nos.: **19-20**
because they relate to subject matter not required to be searched by this Authority, namely:

The technical solutions of claims 19-20 both relate to a method for treating human diseases. Therefore, claims 19-20 do not comply with PCT Rule 39.1(IV).

2. ☐    Claims Nos.:
because they relate to parts of the international application that do not comply with the prescribed requirements to such an extent that no meaningful international search can be carried out, specifically:

3. ☐    Claims Nos.:
because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

| International application No. |
| --- |
| **PCT/CN2024/075125** |

| Patent document cited in search report | | | Publication date (day/month/year) | Patent family member(s) | | | Publication date (day/month/year) |
| --- | --- | --- | --- | --- | --- | --- | --- |
| CN | 111281908 | A | 16 June 2020 | None | | | |
| CN | 112236156 | A | 15 January 2021 | KR | 101966117 | B1 | 05 April 2019 |
| | | | | WO | 2019226015 | A1 | 28 November 2019 |
| | | | | EP | 3804738 | A1 | 14 April 2021 |
| | | | | US | 2021205387 | A1 | 08 July 2021 |
| | | | | IN | 202027055780 | A | 20 August 2021 |
| | | | | JP | 2021524506 | W | 13 September 2021 |
| | | | | EP | 3804738 | A4 | 26 January 2022 |
| | | | | JP | 7214851 | B2 | 30 January 2023 |
| CN | 106955297 | A | 18 July 2017 | US | 2017196926 | A1 | 13 July 2017 |
| | | | | TW | 201725046 | A | 16 July 2017 |
| | | | | CA | 3010907 | A1 | 20 July 2017 |
| | | | | WO | 2017121333 | A1 | 20 July 2017 |
| | | | | US | 9931367 | B2 | 03 April 2018 |
| | | | | AU | 2017206332 | A1 | 26 July 2018 |
| | | | | KR | 20180101460 | A | 12 September 2018 |
| | | | | EP | 3403664 | A1 | 21 November 2018 |
| | | | | JP | 2019501912 | W | 24 January 2019 |
| | | | | ID | 201900738 | A | 01 February 2019 |
| | | | | AU | 2017206332 | B2 | 25 July 2019 |
| | | | | EP | 3403664 | A4 | 18 September 2019 |
| | | | | TW | 678211 | B1 | 01 December 2019 |
| | | | | KR | 102195547 | B1 | 28 December 2020 |
| | | | | CN | 106955297 | B | 12 January 2021 |
| | | | | JP | 6887433 | B2 | 16 June 2021 |
| | | | | EP | 3403664 | B1 | 07 July 2021 |
| | | | | CA | 3010907 | C | 14 March 2023 |
| CN | 112891400 | A | 04 June 2021 | None | | | |
| CN | 1466958 | A | 14 January 2004 | None | | | |
| CN | 114796295 | A | 29 July 2022 | KR | 102327079 | B1 | 16 November 2021 |
| | | | | US | 2022226409 | A1 | 21 July 2022 |
| CN | 1785223 | A | 14 June 2006 | CN | 100353951 | C | 12 December 2007 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310190284

**Non-patent literature cited in the description**

- Guidelines for the Diagnosis and Treatment of Rare Diseases. National Health Commission of China, 2019